# EUROPEAN PATENT SPECIFICATION

(11) **EP 1 595 936 B1**
(45) Date of publication and mention of the grant of the patent: **19.10.2011**
(21) Application number: 04705495.2
(22) Date of filing: 27.01.2004
(51) Int. Cl.: A61K 8/19, A61K 45/06, A61K 47/48, A61Q 19/00

(54) **COMPOSITION FOR EXTERNAL USE**
ZUSAMMENSETZUNG ZUR ÄUSSEREN ANWENDUNG
COMPOSITION A USAGE EXTERNE

(30) Priority: 27.01.2003 JP 2003017866; 20.02.2003 JP 2003086523
(43) Date of publication of application: 16.11.2005
(73) Proprietor: Vitamin C60 Bioresearch Corporation, Tokyo (JP); Ito, Shinobu, Musashimo-shi, Tokyo 180-0002 (JP)
(72) Inventor: ITO, Shinobu, Musashimo-shi, Tokyo 180-0002 (JP); MATSUBAYASHI, Kenji, Tokyo 100-8086 (JP)
(74) Representative: Calamita, Roberto
(86) International application number: PCT/JP2004/000699
(87) International publication number: WO 2004/067678

(56) References cited:
- DE-A1- 4 419 981
- JP-A- 09 278 625
- US-A- 5 612 021
- US-A- 5 994 410
- DATABASE WPI Week 199309 Derwent Publications Ltd., London, GB; AN 1993-071007 XP002437359 & JP 05 017327 A (KANEBO LTD) 26 January 1993 (1993-01-26)
- DATABASE WPI Week 199309 Derwent Publications Ltd., London, GB; AN 1993-071008 XP002437360 & JP 05 017328 A (KANEBO LTD) 26 January 1993 (1993-01-26)
- DATABASE BIOSIS [Online] BIOSCIENCES INFORMATION SERVICE, PHILADELPHIA, PA, US; October 2000 (2000-10), MIYATA NAOKI ET AL: "Reactive species responsible for biological actions of photoexcited fullerenes" XP002437354 Database accession no. PREV200100033479 & YAKUGAKU ZASSHI, vol. 120, no. 10, October 2000 (2000-10), pages 1007-1016, ISSN: 0031-6903
- DATABASE BIOSIS [Online] BIOSCIENCES INFORMATION SERVICE, PHILADELPHIA, PA, US; May 2001 (2001-05), PIOTROVSKY L B ET AL: "Effect of fullerene C60-polyvynilpyrrolidone complexes on influenza virus reproduction" XP002437355 Database accession no. PREV200100443487 & VOPROSY VIRUSOLOGII, vol. 46, no. 3, May 2001 (2001-05), pages 38-42, ISSN: 0507-4088
- DATABASE BIOSIS [Online] BIOSCIENCES INFORMATION SERVICE, PHILADELPHIA, PA, US; April 2002 (2002-04), TSAO NINA ET AL: "In vitro action of carboxyfullerene" XP002437356 Database accession no. PREV200200286102 & JOURNAL OF ANTIMICROBIAL CHEMOTHERAPY, vol. 49, no. 4, April 2002 (2002-04), pages 641-649, ISSN: 0305-7453
- ZEINALOV ET AL: 'Fullerene C60 as an antioxidant for polymers' POLYMER DEGRADATION AND STABILITY vol. 71, no. 2, 2001, pages 197 - 202, XP004295093
- DUGAN ET AL: 'Fullerene-based antioxidants and neurodegenerative disorders' PARKINSONISM AND RELATED DISORDERS vol. 7, no. 3, 2001, pages 243 - 246, XP002979337
- WANG ET AL: 'C60 and Water-Soluble Fullerene Derivatives as Antioxidants Against Radical-Initiated Lipid Peroxidation' J. MED. CHEM. vol. 42, no. 22, 1999, pages 4614 - 4620, XP002979338
- SUSHKO ET AL: 'Static and dynamic light scattering study of strong intermolecular interactions in aqueous solutions of PVP/C60 complexes' POLYMER vol. 43, no. 9, 2002, pages 2769 - 2775, XP004339243

## Description

### Technical Field

The invention of this application relates to a composition for cosmetic skin treatment such as a composition of a formulated skin preparation.

### Background Art

In recent years, carbon nanostructures including fullerenes have attracted attention as the one that brings a new vision to carbon materials, and there has been an increasing interest in their application to medical services and medicines or their application for the purpose of health promotion or the like as well as their application to electronic materials or electrode materials.

For example, blending a fullerene or a fullerene mixture in a cosmetic product for make-up for the purpose of improving dispersibility and coloring property (JP-A-6-192039), a cosmetic composition for sun care by utilizing the UV absorbing effect of a fullerene (JP-A-9-278625), and a method of optically inactivating viruses with the use of a fullerene as a photosensitizer (JP-A-9-322767) have been proposed so far.

However, such an investigation on the use of a fullerenes or its derivative has only just begun, therefore, the fact is that the correlation between the effect of action and the action mechanism or the chemical structure has hardly been elucidated practically. In fact, for example, these points have not been investigated even in the foregoing proposals, therefore, a number of problems for the application of a fullerene or its derivative have still remained.

For example, a composition for external use such as an emulsion, a cream, a lotion, a facial mask, a cleansing agent, an ointment, a dispersion liquid in which ascorbic acid or a derivative thereof, a whitening agent, an antiinflammatory agent, an antioxidant or the like is blended for the purpose of whitening the skin, improving pigmentation, treating acnes, improving wrinkles, improving skin roughness, improving oily skin, improving dry skin, diminishing the appearance of pores, treating scarring, treating flushing of the face, treating hair loss, promoting hair growth, treating a burn injury, sterilizing the skin, killing mites on the skin or improving skin texture has been conventionally used. The antioxidant represented by ascorbic acid derivative has been used in many cases as the active ingredient of a composition for external use since skin aging and skin troubles are caused by free radical damage. In addition, carbon such as charcoal powder or a fullerene has been used in a part of foundations as a UV shielding component.

However, a sufficient effect on whitening the skin, improving pigmentation, treating acnes, improving wrinkles, improving skin roughness, improving oily skin, improving dry skin, diminishing the appearance of pores, treating scarring, treating flushing of the face, treating hair loss, promoting hair growth, treating a burn injury, sterilizing the skin, killing mites on the skin, improving skin texture or the like could not be exerted by applying and using such a conventional antioxidant for a composition for external use. In addition, in the case of using the fullerene, the fullerene is likely to be oxidized due to its potent antioxidative ability, therefore, if it is blended in a composition for external use, there is a problem that it is unstable in a preparation.

In other words, due to the potent antioxidative ability of the fullerene, it exerts a reductive activity in the composition for external use and is rapidly oxidized. As a result, when it is applied to the skin, its antioxidative ability is decreased and weakened, or inactivated, whereby it cannot exert sufficient effect in some cases.

In addition, even if the fullerenes are applied as a preparation for external use, the active ingredient thereof is oxidized and decomposed, and a part of the fullerenes is changed into free radicals on the skin due to the effect of ultraviolet or the like, whereby the effect as an antioxidant is not always exerted sufficiently, and improvement and therapeutic effects as a preparation for external use cannot be exerted sufficiently in some cases.

In addition, there is also a problem that a sufficient effect on whitening the skin, improving pigmentation, treating acnes, improving wrinkles, improving skin roughness, improving oily skin, improving dry skin, diminishing the appearance of pores, treating scarring, treating flushing of the face, treating hair loss, promoting hair growth, treating a burn injury, sterilizing the skin, killing mites on the skin or improving skin texture cannot be obtained with a formulation in which a main component is only the fullerene due to such a problem of oxidization of the fullerene.
Patent literature 1 JP-A-6-192039
Patent literature 2 JP-A-9-278625
Patent literature 3 JP-A-9-322767

Certain fullerenes clathrated in polyvinyl pyrrolidone have been previously disclosed by Sushko et in Polymer, 2002, 43, 2 769 2775, Miyata Naoki et al in Database Biosis no. PREV200100033479, and Piotrovsky et al in Database Biosis no PREV200100443487.

Therefore, the invention of this application makes it an object to overcome the limitation of the conventional arts as above and to provide a technical means of enabling the application of a fullerene and a derivative thereof in various fields relating to biocompatibility to achieve a novel function. In particular, the invention of this application also makes it an object to provide a means for application as a cosmetic product, a formulated skin preparation for external use or the like.

To solve the foregoing problems, the invention of this application firstly provides a composition for cosmetic skin treatment characterized by comprising a fullerene clathrated in polyvinyl pyrrolidone, wherein said composition further comprises at least one kind of the components listed below:
(1) a nonionic surfactant
(2) ascorbic acid or a derivative thereof or a salt thereof, and
(3) an ultraviolet protective agent.

The invention secondly provides the composition for cosmetic skin treatment **characterized in that** the fullerene is C60 fullerene, C70 fullerene or a fullerene mixture containing both.

Further, the invention of this application thirdly provides, regarding the foregoing composition for cosmetic skin treatment **characterized in that** the pH is from 3 to 10, the total concentration of transition metal compounds is 0.1% or less, and at least one kind of components listed below is included:
<1> 0.01% to 50% by weight of a nonionic surfactant listed below,
<2> 0.01% to 20% by weight of an ascorbic acid represented by the following general formula or a derivative thereof or a salt thereof,
<3> 0.001 to 50% by weight of an ultraviolet protective agent listed below, and
<4> 0.01% to 10% by weight of a storing stabilizer or an organic acid having a chelating effect or a salt thereof,
   wherein the nonionic surfactant is at least one kind selected from a POE sorbitan fatty acid ester such as POE sorbitan monooleate, POE sorbitan monostearate, POE sorbitan monolaurate or POE sorbitan tetraoleate, a POE sorbitol fatty acid ester such as POE sorbitol monolaurate, POE sorbitol monooleate, POE sorbitol pentaoleate or POE sorbitol monostearate, a POE glycerin fatty acid ester such as POE glycerin monostearate or POE glycerin triisostearate, a POE fatty acid ester such as POE monooleate, a POE alkyl ether such as POE lauryl ether, a POE alkylphenyl ether such as POE octylphenyl ether, a POE-POP alkyl ether such as POE-POP cetyl ether, a tetra POE-tetra POE ethylenediamine condensate, POE castor oil, a hardened castor oil derivative, a POE beeswax lanolin derivative, an alkanolamide such as lauric monoethanolamide, a POE propylene glycol fatty acid ester, a POE alkylamine, a POE fatty acid amide, a sucrose fatty acid ester, a POE nonylphenyl formaldehyde condensate, an alkyl ethoxy dimethylamine oxide, trioleyl phosphate and a polyglycerin fatty acid ester, wherein the ascorbic acid or the derivative thereof or the salt thereof is a compound represented by the general formula (1) 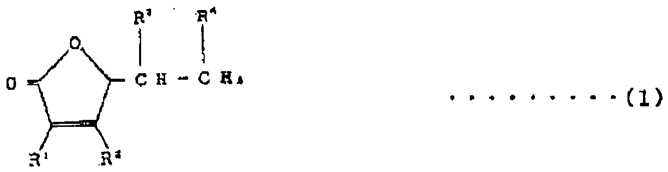
(wherein each of R¹, R², R³ and R⁴ independently denotes a hydroxyl group, an ester group of the hydroxyl group with an inorganic or an organic acid, a glycoside group of the hydroxyl group with a saccharide, a ketal group or an acetal group of adjacent two hydroxyl groups among the hydroxyl groups with a ketone or with an aldehyde, however, R¹ and R² are not a hydroxyl group at the same time) or a salt thereof, or at least one kind selected therefrom, wherein the ultraviolet protective agent is at least one kind selected from a cinnamic acid-based ultraviolet absorbent such as 2-ethylhexyl p-methoxycinnamate, isopropyl p-methoxycinnamate, p-methoxyhydrocinnamate diethanolamine salt, glyceryl mono-2-ethylhexanoate di-p-methoxycinnamate, octyl methoxycinnamate or methyl diisopropylcinnamate, a benzophenone-based ultraviolet absorbent such as 2-hydroxy-4-methoxybenzophenone, 2-hydroxy-4-methoxybenzophenone-5-sulfuric acid, 2-hydroxy-4-methoxybenzophenone-5-sodium sulfate, 2,4-dihydroxy-benzophenone, 2,2'-dihydroxy-4,4'-dimethoxy-benzophenone, 2,2'-dihydroxy-4-methoxybenzophenone, 2,2',4,4'-tetrahydroxybenzophenone or 2-hydroxy-4-n- octoxybenzophenone, a benzoic acid-based ultraviolet absorbent such as p-aminobenzoic acid, ethyl p-aminobenzoate, butyl p-aminobenzoate, 2-ethylhexyl p-dimethylaminobenzoate, glyceryl p-aminobenzoate or amyl p-aminobenzoate, a salicylic acid-based ultraviolet absorbent such as 2-ethylhexyl salicylate, triethanolamine salicylate, homomenthyl salicylate, dipropylene glycol salicylate, methyl salicylate, ethylene glycol salicylate, phenyl salicylate, amyl salicylate, benzyl salycylate, isopropylbenzyl salicylate or potassium salicylate, a dibenzoylmethane-based ultraviolet absorbent such as 4-t-butyl-4'-methoxydibenzoylmethane, 4-isopropyl-dibenzoylmethane, 4-methoxydibenzoylmethane or 4-t-butyl-4'-hydroxydibenzoylmethane, menthyl-O-amino-benzoate, 2-phenyl-bcnzimidazolc-5-sulfuric acid, 2-phenyl-5-methylbenzoxazole, 3-(4-methylbenzilidene)-camphor, 2-ethylhexyl-2-cyano-3,3-diphenylacrylate, 2-ethyl-2-cyano-3,3'-diphenyl-acrylate, 2-(2'- hydroxy-5-methylphenyl)benzotriazole, an anthranilic acid-based ultraviolet absorbent such as menthyl anthranilate, an urocanic acid-based ultraviolet absorbent such as ethyl urocanate, titanium oxide, zirconium oxide, cerium oxide, zinc oxide and a silica-coated metal oxide thereof, and wherein the storing stabilizer or the organic acid having a chelating effect or the salt thereof is at least one kind selected from the group consisting of erythorbic acid and a salt thereof, dibutylhydroxytoluene, tocopherol and a derivative thereof, porphyrin, butylhydroxyanisole, sodium bisulfite, anhydrous sodium sulfite, gallic acid and a derivative thereof, alanine, sodium hydroxyethyl ethylenediamine triacetate, ethylenediamine tetraacetic acid and a salt thereof, citric acid and a salt thereof, gluconic acid, tartaric acid, phytic acid, sodium polyphosphate and sodium metaphosphate.

In addition, the invention of this application fourthly provides a fullerene, composition further including at least one kind of components listed below in the foregoing stable fullerene composition for cosmetic skin treatment, an effect on whitening the skin, improving pigmentation, treating acnes, improving wrinkles, improving skin roughness, improving oily skin, improving dry skin, diminishing the appearance of pores, treating scarring, treating flushing of the face, treating hair loss, promoting hair growth, treating a burn injury, sterilizing the skin, killing mites on the skin or improving skin texture is enhanced:
0.01% to 20% by weight of a whitening component listed below,
0.001 to 10% by weight of an antiinflammatory component, and
0.001 to 10% by weight of an antioxidative component, wherein
the whitening component is at least one kind selected from cysteine, a derivative thereof and a salt thereof, glabridin, glabrene, liquiritin, isoliquiritin, placenta extract, hydroquinone and a derivative thereof, resorcinol and a derivative thereof, and glutathione. The invention fifthly provides the composition for cosmetic skin treatment in which the antiinflammatory component is at least one kind selected from glycyrrhizinic acid, glycyrrhetinic acid, a derivative thereof and a salt thereof, mefenamic acid, phenylbutazone, indomethacin, ibuprofen, ketoprofen, allantoin, guaiazulene, panthenol, a derivative thereof and a salt thereof, ε-aminocapronic acid, diclofenac sodium and tranexamic acid, and sixthly provides the composition for cosmetic skin treatment in which the antioxidative component is at least one kind selected from superoxide dismutase, mannitol, histidine, tryptophan, bilirubin, quercetin, quercitrin, polyphenol, proanthocyanidin, tocotrienol, catechin, a catechin derivative, rutin and a derivative thereof, gallic acid and a derivative thereof, ubiquinone, astaxanthin, carotene, other carotenoids, a derivative thereof and a salt thereof, a vitamin B group, a derivative thereof and a salt thereof, a vitamin D group, a derivative thereof and a salt thereof, a vitamin E group, a derivative thereof and a salt thereof, dibutylhydroxytoluene and butylhydroxyanisole. In a number of preferred embodiments, the composition for cosmetic treatment of the invention is a cream, gel, ointment, emulsion, facial mask, cataplasm, dispersion liquid, solid, paste, mousse or cleansing agent.

According to the invention of this application as above, the limitation of the conventional arts can be overcome, a technical means of enabling the application of a fullerene in various fields relating to biocompatibility to achieve a novel antioxidative function or the like can be provided, in addition, particularly a means for application as a cosmetic product, a formulated skin preparation for external use or the like can be provided.

In addition, in the invention of this application, a composition for cosmetic skin treatment having clear improvement and therapeutic effects on various skin lesions, in which a fullerene is stably maintained without showing the antioxidative activity in the composition for cosmetic skin treatment and it effectively exerts the antioxidative ability only after it is applied to and absorbed in the skin, is provided.

In addition, the fullerene-containing composition of the invention of this application can exert a clear therapeutic effect on whitening the skin, improving pigmentation, treating acnes, improving wrinkles, improving skin roughness, improving oily skin, improving dry skin, diminishing the appearance of pores, treating scarring, treating flushing of the face, treating hair loss, promoting hair growth, treating a burn injury, sterilizing the skin, killing mites on the skin, improving skin texture or the like.

Such a significant effect is based on the findings that the stability of a fullerene-blending preparation for cosmetic skin treatment can be dramatically improved by setting the pH between 3 and 10, setting the total concentration of transition metal compounds to 0.1% or less, and including at least one kind of a storing stabilizer, a nonionic surfactant, an ascorbic acid derivative, an ultraviolet protective agent in the formulation of the fullerene-containing preparation for cosmetic skin treatment, which was obtained as the result of performing intensive studies in order to improve the stability of a fullerene-blending composition for cosmetic skin treatment thereby improving the effect as a preparation for cosmetic skin treatment by the inventors. Further, it is based on the findings that a therapeutic effect on whitening the skin, improving pigmentation, treating acnes, improving wrinkles, improving skin roughness, improving oily skin, improving dry skin, diminishing the appearance of pores, treating scarring, treating flushing of the face, treating hair loss, promoting hair growth, treating a burn injury, sterilizing the skin, killing mites on the skin or improving skin texture, on which only a protective or preventive effect was shown generally in a prescribed area, can be dramatically enhanced by including at least one kind of a whitening component, an antiinflammatory component and an antioxidative component in this formulation.

### Brief Description of the Drawings

Fig. 1 is a graph showing the results of evaluating antioxidative activities against UVB irradiation.
Fig. 2 is a graph showing the results of evaluating antioxidative activities against t-BuOOH.
Fig. 3 is a graph showing the protective effect of PVP with molecular weight of 8000 on cell death against t-BuOOH.
Fig. 4 is a graph showing the protective effect of PVP with molecular weight of 60000 on cell death against t-BuOOH.
Fig. 5 shows photographs showing the foregoing effect of PVP with molecular of weight 60000.
Fig. 6 is a graph showing the protective effect of vitamin C on cell death against t-BuOOH for comparison.
Fig. 7 is a graph showing the protective effect on cell death against UV irradiation in the case of preadministration and total administration.

### Best Mode for Carrying Out the Invention

The invention of this application has characteristics as above, however, hereunder the embodiments will be explained.

In the composition for cosmetic skin treatment such as a skin composition of the invention of this application, the active ingredient or the useful component is a fullerene as described above, and such a composition comprises said fullerene, clathrated in polyvinyl pyrrolidone (PVP) and further comprises at least one kind of the following components:
<1> a nonionic surfactant
<2> ascorbic acid or a derivative thereof or a salt thereof, and
<3> an ultraviolet protective agent.

The fullerene may be any kind such as C60, C70 or a mixture thereof. For example, the fullerene of the invention of this application may be the one in which plural fullerenes are bound through an alkylene chain such as a methylene chain, the one in which an alkylene chain is bound to a carbon atom at a different location in the fullerene skeleton, or the like. As the derivative of the C60 fullerene, 1 to 40 modifying groups may be bound to one fullerene molecule, and for example, as the derivative of the C70 fullerene, 1 to 50 modifying groups may be bound to one fullerene molecule. Such a modifying group may be independently a hydroxyl group, an ester group of the hydroxyl group with an inorganic or an organic acid, a glycoside group thereof with a saccharide, a ketal group of a hydroxyl group with a ketone or an acetal group thereof with an aldehyde. And the fullerene of this application may be such a fullerene-modified compound, a salt thereof or at least one kind selected therefrom. Further, the fullerene of the invention of this application may be C60 fullerene, C70 fullerene or a nanotube fullerene, or it may be a mixture of one or more selected therefrom. In addition, it may be a fullerene including remaining carbon black (soot including a fullerene), which is an unpurified product of the fullerene, and may be a fullerene in which the concentration of carbon black is 0 to 98% by weight.

In addition, with regard to the fullerene- containing oxygen derivative, the one in which an oxygen atom is bound directly to a carbon atom of the fullerene skeleton or through a carbon chain such as an alkylene chain is considered. For example, a hydroxylated fullerene or the like in which hydroxyl groups are directly bound at a hydroxylation rate of about 50/mol fullerene or less is exemplified.

The average molecular weight of the PVP is preferably 2,000 to 100,000 and with regard to the ratio to the fullerene 10/1 or less as a molar ratio is considered.

In the invention of this application, for example, it is effective that a long chain carboxylic acid having 10 or more carbon atoms, an ester thereof or a salt thereof is included together with the foregoing fullerenes as the active ingredient or the useful component. Furthermore, an oil, a surfactant, a pigment, a moisturizer, an excipient, a base, a cell activator as described later or the like may be blended.

The pH, in the case of containing moisture in a fullerene-cantaining composition for cosmetic skin treatment, though it varies depending on the pH of a bulk product of the fullerene, is preferably in the range of 3 to 10 in general because the fullerene and the derivative thereof can be stably blended.

When the pH of a 0.5% by weight aqueous solution of the bulk product of the fullerene, was measured at 20°C. and the numeric value rounded to the nearest integer is n (n is an integer of 0 to 14), in the case where n is from 3 to 10, the pH of the stable composition for cosmetic skin treatment is in the range of n ± 2, and the pH may be adjusted in the range of pH 3 to 10. In addition, when the rounded numeric value of the pH of a 0.5% by weight aqueous solution of the bulk product of the fullerene, at 20°C is n, in the case where n is 3 or less, the pH of the stable fullerene preparation for cosmetic skin treatment may be adjusted in the range of 3 to 4, and in the case where the pH of n is 10 or more, the pH may be adjusted in the range of 9 to 10. In any case, it is preferred that the pH of the stable fullerene-containing composition for cosmetic skin treatment be adjusted in the range of 3 to 10.

In addition, in the case of the invention of this application, as the stabilized composition for cosmetic skin treatment of pH 3 to 10, it is more desirable that the total concentration of transition metal compounds to be included or contaminated be 0.1 % or less.

In general, the upper limit of a heavy metal concentration has been generally defined for cosmetic materials for the purpose of ensuring safety, and generally there is a restriction on the arsenic content of 10 ppm or less. Meanwhile, it is normal that the upper limit of the concentration of a transition metal has not been generally defined because its safety is relatively high. However, with regard to the fullerene-containing composition of the invention of this application, the knowledge that there is a possibility of leading to a big problem with the safety even if the concentration of the contained transition metals is low is considered. In fact, there is a case where a large amount of a transition metal such as iron may be contaminated in a glass container to be used for packing a cosmetic product, water to be used in a cosmetic material, or a material to be used in many cases in a cosmetic product such as titanium oxide, zinc oxide or diatomaceous earth, and the case where such a transition metal significantly reduces the stability of the fullerene has been confirmed.

With regard to the invention of this application, as further described below, the activity thereof corresponding to the following Examples, the composition formulation to be considered and the like will be explained in detail.

### <A> Activity of fullerenes

### 1. Activity of scavenging hydroxyl radical generated by transition metal ion

When hydrogen peroxide is mixed with ferrous sulfate, so called the Fenton reaction is initiated, thereby generating one of the oxygen radicals, hydroxyl radical, which is generated anywhere in the body in the same way to lead to oxidative damage to DNAs, proteins or lipids, whereby cell death is considered to be caused. Hydroxyl radical generated in this reaction can be effectively scavenged by the fullerene of the invention of this application. In fact, hydroxyl radical scavenging activity of the fullerene of this invention is equal to or more than that of ascorbyl-2-O-phosphate ester which is a pro-vitamin C.

The hydroxyl radical scavenging activity of the fullerene of the invention of this application is not limited to the scavenging activity against hydroxyl radical generated by a transition metal ion, but the fullerene scavenges hydroxyl radical widely generated in vivo or in the skin under various conditions.

Hydroxyl radical scavenging activity has an effect of protecting DNA breakage, DNA damage, cell membrane breakage or cell death, which is caused by superoxide anion radical.

### 2. Activity of scavenging superoxide anion radical generated by enzymatic reaction

It is considered that superoxide anion radical is generated by stagnation of blood flow in the skin or in the process of skin lesions to lead to oxidative damage to DNAs, proteins or lipids thereby causing cell death. Superoxide anion radical is generated by mixing hypoxanthine with xanthine oxidase, which can be effectively scavenged by the fullerene of the invention of this application.

The superoxide anion radical scavenging activity of the fullerene of this application is superior to that of ascorbyl-2-O-phosphate ester which is a pro-vitamin C.

The superoxide anion radical scavenging activity of the fullerene of this application is not limited to the scavenging activity against hydroxyl radical generated by an enzymatic reaction, but the fullerene scavenges superoxide anion radical widely generated in vivo or in the skin under various conditions.

The superoxide anion radical scavenging activity has an effect of protecting DNA breakage, DNA damage, cell membrane breakage or cell death, which is caused by superoxide anion radical. 3. Activity of suppressing peroxide/hydrogen peroxide generated in skin cells by ultraviolet

When the skin is irradiated with sunlight, peroxide/hydrogen oxide is generated in cells by ultraviolet-B in sunlight, or cell death is caused by being subjected to DNA breakage, DNA damage or cell membrane breakage.

When the fullerene of the invention of this application is prescribed in advance before the irradiation, the amount of generated peroxide/hydrogen peroxide will be significantly suppressed. The peroxide/hydrogen peroxide scavenging activity of the fullerene of this invention is equal to or more than that of ascorbyl-2-O-phosphate ester which is a pro-vitamin C.

Since peroxide/hydrogen peroxide penetrates cell membrane and the remaining lifetime thereof is long, it becomes a main cause leading to cell damage, however, the fullerene of the invention of this application not only scavenges peroxide/hydrogen peroxide generated by ultraviolet rays but also scavenges peroxide/ hydrogen peroxide widely generated in vivo or in the skin under various conditions.

The peroxide/hydrogen peroxide scavenging activity of the fullerene of this application has an effect of protecting DNA breakage, DNA damage, cell membrane breakage or cell death, which is caused by peroxide/hydrogen peroxide.

### 4. Activity of suppressing peroxide/hydrogen peroxide generated in skin cells by lipid peroxide

Lipids present in the skin are susceptible to oxidization at any time, whereby it becomes a cause of leading to the death of skin cells. In particular, ceramide or squalene, which is a lipid in the stratum corneum is subjected to oxidization and changed into a hydroperoxide to cause cell death.

When the fullerene of the invention of this application is prescribed in advance, the amount of generated peroxide/hydrogen peroxide will be significantly suppressed. The peroxide/hydrogen peroxide scavenging activity of the fullerene of the invention of this application is equal to or more than that of ascorbyl-2-O-phosphate ester which is a pro-vitamin C.

The fullerene of the invention of this application not only scavenges peroxide/hydrogen peroxide generated by lipid peroxide but also scavenges peroxide/hydrogen peroxide widely generated in vivo or in the skin under various conditions.

The peroxide/hydrogen peroxide scavenging activity of the fullerene of the invention of this application has an effect of protecting DNA breakage, DNA damage, cell membrane breakage or cell death, which is caused by peroxide/hydrogen peroxide.

### 5. Various beautifying skin effects and skin protective effects

The fullerene derivative has an activity of scavenging various oxygen radicals, therefore, it exerts various beautifying skin effects of protecting ultraviolet-induced damage, lipid peroxide-induced damage, ischemic reperfusion injury, melanogenesis, formation of wrinkles/ dullness skin/ sagging skin or cellulite formation which is caused or promoted, or the repair of which is disturbed by oxygen radicals.

By application as a composition for external use, a therapeutic effect on whitening the skin, improving pigmentation, treating acnes, improving wrinkles, improving skin roughness, improving oily skin, improving dry skin, diminishing the appearance of pores, treating scarring, treating flushing of the face, treating hair loss, promoting hair growth, treating a burn injury, sterilizing the skin, killing mites on the skin or improving skin texture is dramatically enhanced.

In addition, in the invention of this application, as the composition for external use per se, it is stably maintained without showing the antioxidative activity and it effectively exerts the antioxidative ability only after it is applied to and absorbed in the skin, and expresses clear improvement and therapeutic effects on various skin lesions.

Accordingly, the composition for external use of the invention of this application can exert a clear therapeutic effect on whitening the skin, improving pigmentation, treating acnes, improving wrinkles, improving skin roughness, improving oily skin, improving dry skin, diminishing the appearance of pores, treating scarring, treating flushing of the face, treating hair loss, promoting hair growth, treating a burn injury, sterilizing the skin, killing mites on the skin, improving skin texture or the like.

That is, the composition is effective for a skin protective effect of protecting all the skin inflammations and subcutaneous inflammations, or promoting wound healing as well as a beautifying skin effect. In particular, it is effective for a bacterial infectious disease, an infectious disease caused by a bacterium, fungus or virus, an infectious disease caused by a parasite, a burn injury, a sunburn, an abrasion, a bruise, an inflammation caused by wound of a bite injury or the like.

### <B> Method of administration of fullerenes as preparation for external use

### 1. Amount of administration

The concentration of the fullerene of the invention of this application may be 0.00001% to 30% by weight, however, from the viewpoint of sense of feeling of use, it is preferably 5% or less. In the case of administration to the skin, with regard to the amount of the composition for external use, it is desirable that 0.001 to 20 ml of a liquid, preferably 0.01 to 5.0 ml per 1 square meter of skin area be applied for external use, fomented or sprayed.

### 2. Form of administration

As an example of the form of the external composition for the skin, it is not particularly limited, and it can be in any form for an external preparation including a water-soluble preparation, an ointment, an emulsion, a cream, a gel, a facial mask, a bath preparation, a cleansing agent, a cataplasm, a dispersion liquid and the like. In addition, the dosage form is not particularly limited, and it can be in a form of solid, paste, mousse, gel, powder, a solution system, a solubilization system, an emulsification system, a powder dispersion system, or a multilayer form. Particularly for an aqueous solution, an emulsion, an ointment, a gel, a water soluble preparation, a serum or a facial mask, by using a humidifier, a pulse introduction apparatus, an ion introduction apparatus, a sonic wave introduction apparatus or a magnetic wave introduction apparatus after such a preparation is used externally, penetration of the fullerene into the skin can be promoted whereby a larger effect can be exerted.

With regard to a method of application, in the case of a liquid preparation, all the physically possible methods such as spraying, patching, fomenting, dipping and masking can be used.

### <C> External preparation containing fullerene

The antioxidative composition and the composition for external use of the invention of this application are achieved basically as a combination of various components constituting conventionally known cosmetic product or preparations for external use.

Hereunder, first, the general of such a component will be outlined.

### 1. Oil

It is preferred that the fullerene of the invention of this application be administered in vivo, particularly to the skin by dispersing it into an oil, preferably a natural oil, more preferably an oil containing one or more kinds of oils selected from orange oil, beaver oil, olive oil and pine oil.

As the oil, any oil such as a hydrocarbon, a wax, a fatty acid, a higher alcohol, a ester oil, a silicone oil or a fluorinated oil can be used whether it be a natural oil or a synthetic oil, or regardless of the property such as solid, semisolid or liquid, provided that it has been used in a common cosmetic material. Examples include hydrocarbons such as squalane, squalene, ceresin, paraffin, paraffin wax, liquid paraffin, pristane, polyisobutylene, microcrystalline wax and vaseline, waxes such as beewax, carnauba wax, candelilla wax and whale wax, animal oils such as beef tallow, beef foot oil, beef bone fat, hydrogenated beef tallow, hydrogenated oil, turtle oil, lard, horse fat, mink oil, liver oil and egg yolk oil, lanolin derivatives such as lanolin, liquid lanolin, reduced lanolin, lanolin alcohol, hard lanolin, lanolin acetate, lanolin fatty acid isopropyl, POE lanolin alcohol ether, POE lanolin alcohol acetate, polyethylene glycol lanolin fatty acid and POE hydrogenated lanolin alcohol ether, and fatty acids such as lauric acid, myristic acid, palmitic acid, stearic acid, behenic acid, undecylenic acid, oleic acid, arachidonic acid, docosahexaenoic acid (DHA), isostearic acid, and 12-hydroxystearic acid.

In addition, examples of the oil include higher alcohols such as lauryl alcohol, myristyl alcohol, palmityl alcohol, stearyl alcohol, behenyl alcohol, hexadecyl alcohol, oleyl alcohol, isostearyl alcohol, hexyldodecanol, octyldodecanol, cetostearyl alcohol, 2-decyltetradecinol, cholesterol, phytosterol, sitosterol, lanosterol, POE cholesterol ether, mono stearyl glycerin ether (batyl alcohol), and ester oils such as diisobutyl adipate, 2-hexyl decyl adipate, di-2-heptyl undecyl adipate, N-alkyl glycol monoisostearate, isocetyl isostearate, trimethylolpropane triisostearate, di-2-ethylhexane acid ethylene glycol ester, 2-ethylhexane acid cetyl ester, tri-2-ethylhexane acid trimethylolpropane ester, tetra-2-ethylhexane acid pentaerythritol ester, cetyl octanoate, octyldodecyl gum ester, oleyl oleate, octyldodecyl oleate, decyl oleate, neopentyl glycol dicaprate, triethyl citrate, 2-ethylhexyl succinate, amyl acetate, ethyl acetate, butyl acetate, isocetyl stearate, butyl stearate, diisopropyl sebacate, di-2-ethylhexyl sebacate, cetyl lactate, myristyl lactate, isopropyl palmitate, 2-ethylhexyl palmitate, 2-hexyl decyl palmitate, 2-heptyl undecyl palmitate, 12-hydroxy stearyl acid cholesteryl, dipentaerythrytol fatty acid ester, isopropyl myristate, octyldodecyl myristate, 2-hexyl decyl myristate, myristyl myristate, dimethyl octanoic acid hexyl decyl, ethyl laurate, hexyl laurate, N-lauroyl-L-glutamic acid 2-octyldodecyl ester and diisostearyl malate. In addition, examples of the oil include glyceride oils such as acetoglyceride, triisooctanoic acid glyceride, triisostearic acid glyceride, triisopalmitic acid glyceride, tri-2-ethyl hexanoic acid glyceride, monostearic acid glyceride, di-2-heptyl undecanoic acid glyceride and trimyristic acid glyceride, higher alkoxy-modified silicones such as dimethyl polysiloxane, methylphenyl polysiloxane, methylhydrogen polysiloxane, octamethyl cyclotetrasiloxane, decamethyl cyclopentasiloxane, dodecamethyl cyclohexasiloxane, tetramethyl tetrahydrogen cyclotetrasiloxane and stearoxysilicone, silicone oils such as higher fatty acid-modified silicones, silicone resins, silicone rubbers and silicone oils, and fluorinated oils such as perfluoropolyether, perfluorodecalin and perfluorooctane.

### 2. Surfactant

In a preparation for a cosmetic product containing the fullerene of the invention of this application, caprylic acid monoglyceride and/or capric acid monoglyceride can be included, and further lauric acid monoglyceride can be blended. All caprylic acid monoglyceride, capric acid monoglyceride and lauric acid monoglyceride (hereinafter referred to as merely glyceride in some cases) have been designated as a food additive, a glycerin fatty acid ester, the safety of which has been confirmed, and are a food emulsifier harmless to eat.

In the fullerene of the invention of this application, an emulsifier can be added in order to disperse it in water. As the emulsifier, for example, a nonionic surfactant such as polyoxyethylene sorbitan fatty acid ester such as polyoxyethylene sorbitan monooleic acid ester having an HLB value of 10 or more, or polyglycerin fatty acid ester, or an anionic surfactant such as sodium lauryl sulfate can be used.

As a surfactant, an anionic, a cationic, a nonionic or an amphoteric active agent is used. Examples of the anionic surfactant include fatty acid soaps such as sodium stearate and triethanolamine palmitate, alkyl ether carboxylic acids and salts thereof, carboxylate salts such as amino acid-fatty acid condensates, alkylsulfonic acids or alkenesulfonates, fatty acid ester sulfonates, fatty acid amide sulfonates, sulfonates of alkyl sulfonates and their formalin condensates, alkyl sulfate ester salts, higher secondary alcohol sulfate ester salts, alkyl and aryl ether sulfate ester salts, fatty acid ester sulfate ester salts, fatty acid alkylolamide sulfate ester salts, sulfate ester salts such as Turkey red oil, alkyl phosphates, ether phosphates, alkyl aryl ether phosphates, amide phosphates, N-acylamino acid type active agent and the like.

Examples of the cationic surfactant include amine salts such as alkylamine salts, polyamines and aminoalcohol fatty acid derivatives, quaternary alkylammonium salts, aromatic quaternary ammonium salts, pyridium salts, imidazolium salts and the like. Examples of the nonionic surfactant include sorbitan fatty acid esters, glycerin fatty acid esters, polyglycerin fatty acid esters, propylene glycol fatty acid esters, polyethylene glycol fatty acid esters, sucrose fatty acid esters, polyoxyethylene alkyl ethers, polyoxypropylene alkyl ethers, polyoxyethylene alkyl phenyl ethers, polyoxyethylene fatty acid esters, polyoxyethylene sorbitan fatty acid esters, polyoxyethylene sorbitol fatty acid esters, polyoxyethylene glycerin fatty acid esters, polyoxyethylene propylene glycol fatty acid esters, polyoxyethylene castor oil, polyoxyethylene hydrogenated castor oil, polyoxyethylene phytostanol ether, polyoxyethylene phytosterol ether, polyoxyethylene cholestanol ether, polyoxyethylene cholesteryl ether, polyoxyalkylene-modified organopolysiloxanes, polyoxyalkylene/ alkyl comodified organopolysiloxanes, alkanolamides, sugar ethers, sugar amides and the like. Examples of the amphoteric surfactant include betaine, aminocarboxylates, imdazoline derivatives and the like.

Examples of a metal soap include aluminum 12-hydroxystearate, zinc stearate, aluminum stearate, calcium stearate, magnesium stearate, zinc myristate, magensium myristate, zinc cetylphosphate, calcium cetylphosphate, zinc sodium cetylphosphate, zinc laurate, zinc undecylenate and the like.

### 3. Pigment

Since many of the fullerenes of the invention of this application are colored, it is desired to adjust the hue to comfortable level as a cosmetic product by blending a pigment as needed. Examples of a colored pigment include inorganic red pigments such as iron oxide, iron hydroxide and iron titanate, inorganic brown pigments such as γ-iron oxide, inorganic yellow pigments such as iron oxide yellow and loess, inorganic black pigments such as iron oxide black and carbon black, inorganic violet pigments such as manganese violet and cobalt violet, inorganic green pigments such as chromium hydroxide, chromium oxide, cobalt oxide and cobalt titanate, inorganic blue pigments such as Prussian blue and ultramarine blue, lakes of tar pigments, lakes of natural pigments, powder complexes in which powders as above are complexed and the like. Examples of a pearl pigment include titanium oxide-coated mica, titanium oxide-coated mica, bismuth oxychloride, titanium oxide-coated bismuth oxychloride, titanium oxide-coated talc, fish scales, titanium oxide-coated colored mica and the like. Examples of a metallic powder pigment include aluminum powder, copper powder, stainless powder and the like.

Examples of the tar pigment include Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206, Orange No. 207 and the like. Examples of the natural pigment include carminic acid, laccaic acid, carthamin, bradilin, crocin and the like. The foregoing powders such as an inorganic powder, an organic powder, a pigment and a tar pigment may also be complexed or surface-treated with an oil, a silicone or a fluoride compound.

### 4. Moisturizer

A polyhydric alcohol such as propylene glycol, glycerin, polyglycerin, sorbitan or sorbitol is added in order to moisturize the skin and to alleviate irritation. Examples of the moisturizer include simple alkali hot spring water, deep-sea water, mucopolysaccharides such as hyaluronic acid, chondroitin sulfate, dermatan sulfate, heparan sulfate, heparin and keratan sulfate and salts thereof, proteins such as collagen, elastin, and keratin, derivatives thereof and salts thereof, phospholipids derived from soybeans or eggs, glycolipids, ceramide, mucin, honey, erythritol, maltose, maltitol, xylitol, xylose, pentaerythritol, fructose, dextrin and derivatives thereof, saccharides such as mannitol, sorbitol, inositol, trehalose and glucose, urea, asparagine, aspartic acid, alanine, arginine, isoleucine, ornithine, glutamine, glycine, glutamic acid, derivatives thereof and salts thereof, cycteine, cystine, citrulline, threonine, serine, tyrosine, tryptophan, theanine, valine, histidine, hydroxylysine, hydroxyproline, pyrrolidone carboxylic acid and salts thereof, amino acids such as proline, phenylalanine, methionine and lysine, derivatives thereof and salts thereof, and the like.

Further, examples of the moisturizer include D-panthenol, avocado extract, almond oil, locust bean extract, rice extract, strawberry extract, fennel extract, Malva sylvestris extract, Coptis extract, olive oil, Lamium extract, cacao butter, oat grass extract, Hedera extract, Sasa veitchii extract, Gardenia jasminoides extract, grape fruit extract, Geranium extract, gentian extract, burdock extract, Clematis apiifolia extract, sesame extract, cactus extract, Saponaria officinalis extract, ginger extract, Rehmannia glutinosa extract, shea butter, Filipendula extract, Cnidium extract, Malva sylvestris extract, Thymus vulgaris extract, Camellia extract, corn extract, Cordyceps Sinensis extract, Potentilla tormentilla extract, Houttuynia cordata extract, Ophiopogon japonicus extract, Lupinus perennis extract, Hamamelis virginiana extract, Mentha extract, green Mentha extract, western Mentha extract, parsley extract, rose extract, sunflower extract, Hinoki extract, sponge gourd extract, prune extract, butcher's broom extract, borage oil, peony extract, jojova oil, lime tree extract, hop extract, pine extract, Silybum marianum extract, macadamia nut extract, Cydonia oblonga extract, Lithospermum erythrorhizon extract, meadowfoam oil, Melissa extract, Centaurea cyanus extract, Lily extract, Yuzu extract, lime extract, Lavender extract, Gentiana scabra extract, Sanguisorba officinalis extract, apple extract and the like. One or more kinds of the moisturizers described above can be selected as needed and blended.

### 5. Excipient and base

Examples of a gelling agent include amino acid derivatives such as N-lauroyl-L-glutamic acid and α, γ-di-n-butylamine, dextrin fatty acid esters such as dextrin palmitic acid ester, dextrin stearic acid ester and dextrin 2-ethylhexaminic acid palmitic acid ester, sucrose fatty acid esters such as sucrose palmitic acid ester and sucrose stearic acid ester, benzylidene derivatives of sorbitol such as monobenzylidene sorbitol and dibenzylidene sorbitol, clay minerals modified with organic compounds such as dimethylbenzyldodecyl ammonium montmorillonite clay and dimethyldioctadecyl ammonium montmorillonite clay.

Examples of an alcohol include lower alcohols such as ethanol and isopropanol, polyhydric alcohols such as glycerin, diglycerin, ethylene glycol, diethylene glycol, triethylene glycol, propylene glycol, dipropylene glycol, 1,3-butylene glycol and polyethylene glycol, and the like.

Examples of a water-soluble polymer include polymers derived from plants such as gum arabic, tragacanth, galactan, carob gum, guar gum, karaya gum, carrageenan, pectin, agar, algae-colloid, tranto gum, locust bean gum and galactomannan, polymers derived from microorganisms such as xanthan gum, dextran, succinoglucan and pullulan, polymers derived from animals such as casein, albumin and gelatin, starch polymers such as starch, carboxymethyl starch and methylhydroxypropyl starch, cellulose polymers such as methylcellulose, ethylcellulose, methylhydroxypropylcellulose, carboxymethylcellulose, hydroxymethylcellulose, hydroxypropylcellulose, nitrocellulose, sodium cellulose sulfate, sodium carboxymethylcellulose, crystalline cellulose and cellulose powder, alginate polymers such as sodium alginate and propylene glycol alginate, vinyl polymers such as polyvinyl methyl ether, carboxyvinyl polymer and alkyl-modified carboxyvinyl polymer, polyoxyethylene polymers, polyoxyethylene/ polyoxypropylene copolymers, acrylic polymers such as sodium polyacrylate, polyethyl acrylate and polyacrylamide, inorganic water-soluble polymers such as polyethyleneimine, cation polymers, bentonite, laponite and hectorite, and the like. In addition, among these, film-forming agents such as polyvinyl alcohol and polyvinyl pyrrolidone are included.

As a powder, any member such as an inorganic powder, an organic powder or a pigment can be used regardless of the shape (such as spherical, acicular or platy form), the grain size (the order of such as fume, fine grain or pigment), or the grain structure (such as porous or nonporous), provided that it has been used in a common cosmetic material. Examples of the inorganic powder include magnesium oxide, barium sulfate, calcium sulfate, magnesium sulfate, calcium carbonate, magnesium carbonate, talc, synthetic mica, mica, kaolin, sericite, white mica, synthetic mica, phlogopite, ruby mica, biotite, lithia mica, silicic acid, silicic acid anhydride, aluminum silicate, magnesium silicate, aluminum magnesium silicate, sulfur-containing aluminum silicate, calcium silicate, barium silicate, strontium silicate, metal salts of tungstic acid, hydroxyapatite, vermiculite, haidilite, montmorillonite, zeolite, ceramics powder, calcium secondary phosphate, alumina, aluminum hydroxide, boron nitride, boron nitride and the like.

Examples of the organic powder include polyamide powder, polyester powder, polyethylene powder, polypropylene powder, polystyrene powder, polyurethane, benzoguanamine powder, polymethyl benzoguanamine powder, tetrafluoroethylene powder, polymethylmethacrylate powder, silk powder, nylon powder, 12-nylon, 6-nylon, styrene-acrylic acid copolymer, divinylbenzene-styrene copolymer, vinyl resin, urea resin, phenol resin, fluororesin, silicone resin, acrylic resin, melamine resin, epoxy resin, polycarbonate resin, microcrystalline fiber powder, lauroyl lysine and the like. 6. Cell activator

In order to promote the drug efficacy of the fullerene of the invention of this application when it is administered in vivo or in the skin, it is preferred that a drug for activating cells that are objects of administration be administered at the same time. Examples of the cell activator include nucleic acid-related substances such as deoxyribonucleic acids and salts thereof, adenylic acid derivatives such as adenosine triphosphate, adenosine monophosphate and salts thereof, ribonucleic acids and salts thereof, cyclic AMP, cyclic GMP, flavin adenine nucleotide, guanine, adenine, cytosine, thymine, xanthine, caffeine, which is a derivative thereof, theophylline, and salts thereof, calf blood extract solution, serum protein-free extract, spleen extract, egg ingredients of birds and the like, cock's crest extract, shell extract, shell fish meat extract, royal jelly, silk protein, decomposition products thereof and derivatives thereof, hemoglobin and decomposition products thereof, lactoferrin and decomposition products thereof, mollusca extract such as squid ink, fish meat extract, extracts derived from animals such as mammals, birds, shell fish, insects, fish, molluscs and crustaceas, extracts derived from microorganisms selected from fermentation and metabolic products such as yeast extract, lactic acid bacteria extract and bifidobacteria extract.

Further, examples of the cell activator include a vitamin A group such as retinol and derivatives thereof (such as retinol palmitate and retinol acetate), retinal and derivatives thereof, dehydroretinal, tretinoin and carotenoids such as carotene, a vitamin B group such as thiamines (thiamine hydrochloride, thiamine sulfate), riboflavins (such as riboflavin and riboflavin acetate), pyridoxines (such as pyridoxine hydrochloride and pyridoxine dioctanoate), flavin adenine nucleotide, cyanocobalamin, folic acids, nicotinic acids (such as nicotinic acid amide and nicotinic acid benzyl) and cholines, apricot extract, ginkgo extract, Panax ginseng extract, barley extract, orange extract, cucumber extract, kiwi extract, Lentinus Edodes extract, Equisetum extract, Swertia extract, Zizyphi fructus extract, capsicum extract, garlic extract, carrot extract, Poria cocos extract, peach extract, lettuce extract, lemon extract, Ganoderma lucidum extract, rosemary extract, asparagus extract, Polygonum bistorta extract, Pisum sativum extract, Rose Fruit extract, Scutellaria root extract, ononis extract, seaweed extract, raspberry extract, Sophorae Radix extract, Millettia extract, Periploca sepium extract, plant oil containing linoleic acid, Asarum extract, Crataegus cuneata extract, Cassia nomame extract, Lilium extract, peony root extract, Inula britannica extract, White Mulberry Root-bark extract, soybean extract, tea extract, Angelica acutiloba extract, molasses extract, Ampelopsis japonica extract, beech tree extract, grape seed extract, Flor de Manita extract, hop extract, Rosa rugosa extract, Pseudocydonia sinensis extract, Saxifraga stolonifera extract, Coix extract, Momordicae grosvenori extract, in addition, extracts of Rubia tinctorum, red grape, Mallotus japonicus, Akebia quinata, hemp, morning glory, red bean, Leguminosae, Hydrangea macrophylla, Gynostemma pentaphyllum, Polygonum cuspidatum, fig, Ginkgo, ilang-ilang, Prunella vurgaris, Japanease plum, uva-ursi, Citrus unshiu, Acanthopanax senticosus, Cassia tora, Sophora japonica, Pisum sativum, Plantago asiatica, gumbo, Inula britannica, Juglans mandshurica, Patrinia scabioaefoia, garden strawberry, Japanese persimmon, Glechoma hederacea, cashew, Valeriana fauriei, Trichosanthes cucumeroides, Chinese quince, guarana, Platycodon grandiflorum, chrysanthemum, Catalpa ovata, Rumex japonicus, Gymnema sylvestre, Agrimonia pilosa, guava, Lycium chinense, Pueraria lobata, Cinnamomum camphora, Japanese common chestnut, Millettia extract, Laurus nobilis, cinnamon bark, Rubus chingii, Piper nigrum, coffee, Scrophularia buergeriana, Colombo, Camellia sasanqua, Zanthoxylum piperitum, saffron, Prunus, pomegranate, Sophora subprostrata root, Cassia nomame, Aster tataricus, Acorus calamus, watermelon, stevia, prune, English ivy, pear, Achillea millefolium, Juniperus communis, horseradish, Acorus gramineus, Japanese parsley, Senegae Radix, Senna angustifolia, Rheum palmatum, Citrus aurantium, Tamarindus indica, Aralia elata, dandelion, chicory, clove, Schisandra chinensis, Polyporus umbellatus, Oenothera tetraptera, Centella asiatica, Commelina communis, Tetragonia tetragonoides, Juglans regia, Benincasa hispida, Eucommia ulmoildes, Abelmoschus manihot, Capsella bursa-pastoris, Citrus natsudaidai, Nandina domestica, Picrasma quassioides, Achillea alpina, pineapple, hibiscus, papaya, basil, lotus, naked barley, Belamcanda chinensis, peanut, Plectranthus japonicus, Trapa japonica, pistachio, Thujopsis dolabrata, Agaricus blazei Murrill, Angelica dahurica, Eriobotrya japonica, Tussilago farfara, Rhus javanica, Eupatorium japonicum, blueberry, Ledebouriella seseloides, Physalis alkekengi, Magnolia hypoleuca, Chaenomeles speciosa, Rosa rugosa, ephedra sinica, mango, Ganoderma lucidum, Bupleurum falcatum, Lythrum anceps, Cryptotaenia japonica, Acacia baileyana, Melilotus officinalis, melon, Magnolia quinquepeta, Momordica charantia, grosvenori, Corchorus olitorius, bean sprout, Alpiniae oxyphyllae, Leonurus sibiricus, Rodgersia podophylla, palm, Yashajitsu, Viscum album, Polygonum hydropiper, Phytolacca esculenta, Myrica rubra, Daphniphyllum macropodum, Artemisia princeps, rye, orchid, Euphoria longana, apple, lychee, Forsythia suspensa and the like, extracts derived from plants such as hinokitiol and cepharanthin, α- and γ-linolenic acid, eicosapentaenoic acid and derivatives thereof, estradiol, derivatives thereof and salts thereof, organic acids such as glycolic acid, succinic acid, lactic acid and salicylic acid, derivatives thereof and salts thereof and the like. One or more kinds of the cell activators described above can be selected as needed and blended.

Examples of a vitamin include a vitamin K group such as phytonadione, menaquinone, menadione and menadiol, a vitamin P group such as eriocitrin and hesperidin, biotin, cartinine, ferulic acid, and the like. Examples of a blood circulation promoter include nonylic acid vanillylamide, capsaicin, zingerone, Cantharides tincture, ichthammol, α-borneol, inositol hexanicotinate, cyclandelate, cinnarizine, tolazoline, acetylcholine, verapamil, γ-olizanol and the like. Examples of a skin astringent include tannic acid and the like, examples of an antiseborrheic agent include thiantol and the like, and examples of an enzyme include lipase, papain and the like.

Examples of an amino acid include amino acids such as glycine, alanine, valine, isoleucine, serine, threonine, aspartic acid, glutamic acid, asparagine, glutamine, lysine, hydroxylysine, arginine, cystine, methionine, phenylalanine, tyrosine, proline, hydroxyproline, ornithine, citrulline and theanine, derivatives thereof and salts thereof, amino acid derivatives such as pyrrolidone carboxylic acid and the derivatives thereof and the like. Examples of the nucleic acid-related substance include deoxyribonucleic acids and salts thereof, adenylic acid derivatives selected from adenosine triphosphate, adenosine diphosphate, adenosine monophosphate and salts thereof, ribonucleic acids and salts thereof, cyclic AMP, cyclic GMP, flavin adenine nucleotide, guanine, adenine, cytosine, thymine, xanthine, caffeine, which is a derivative thereof, theophylline and salts thereof, and examples of a hormone include estradiol, ethinylestradiol and the like.

### 7. Ascorbic acid

In the composition for external use of the invention of this application, an ascorbic acid or a derivative thereof is preferably used.

In particular, it may be the compound represented by the foregoing formula, the salt thereof or at least one kind selected therefrom.

The ascorbic acid can also be used as it is, and the ascorbic acid may be in any of an L-form, D-form, and a DL-form, or may be used as such as an ester with an inorganic acid or an organic acid, as a glycoside with a saccharide, or as a ketal or an acetal in which two adjacent hydroxyl groups among the hydroxyl groups of the ascorbic acid are bound to a ketone or an aldehyde.

Examples of the inorganic acid in this case include phosphoric acid, diphosphoric acid, triphosphoric acid, sulfuric acid and the like, and phosphoric acid is preferred. Examples of the organic acid include acetic acid, propionic acid, butyric acid, isobutyric acid, stearic acid, myristic acid, palmitic acid and the like, and a higher fatty acid such as palmitic acid is particularly preferred. Examples of the saccharide include glucose, sucrose, fructose and the like, and glucose is particularly preferred. Examples of the ketone include acetone and methyl ethyl ketone, and examples of the aldehyde may include acetic aldehyde, propynoic aldehyde, benzoic aldehyde and the like. When it is used as a salt, examples may include sodium, potassium, magnesium, calcium and the like, and a sodium salt and a magnesium salt are particularly preferred.

Specific examples of such ascorbic acid derivative include, for example, derivatives of ascorbic acid esters, L-ascorbic acid alkyl esters, L-ascorbic acid phosphate esters, L-ascorbic acid sulfate esters and the like such as ascorbyl-2-phosphate, ascorbyl-2-diphosphate, ascorbyl-2-triphosphate, ascorbyl-2-polyphosphate, ascorbyl-2-phosphate diesters, ascorbyl-2-phosphate-6-palmitate, ascorbyl-2-phosphate-6-myristate, ascorbyl-2-phosphate-6-stearate, ascorbyl-2-phosphate-6-oleate, ascorbyl-2-glucoside, ascorbyl-2-glucoside-6-palmitate, ascorbyl-2-glucoside-6-myristate, ascorbyl-2-glucoside-6-stearate, ascorbyl-2-glucoside-6-oleate and ascorbyl-2-sulfate, and may include salts thereof such as alkali metal salts including sodium salts, potassium salts and the like, and alkaline earth metal salts including calcium salts, magnesium salts and the like. More specific examples include L-ascorbic acid palmitate, L-ascorbic acid dipalmitate, L-ascorbic acid isopalmitate, L-ascorbic acid diisopalmitate, L-ascorbic acid tetraisopalmitate, L-ascorbic acid stearate, L-ascorbic acid distearate, L-ascorbic acid isostearate, L-ascorbic acid diisostearate, L-ascorbic acid myristate, L-ascorbic acid dimyristate, L-ascorbic acid isomyristate, L-ascorbic acid diisomyristate, L-ascorbic acid oleate, L-ascorbic acid dioleate, L-ascorbic acid-2-ethylhexanoate, L-ascorbic acid phosphate ester sodium, L-ascorbic acid phosphate ester potassium, L-ascorbic acid phosphate ester magnesium, L-ascorbic acid phosphate ester calcium, L-ascorbic acid phosphate ester aluminum, L-ascorbic acid sulfate ester sodium, L-ascorbic acid sulfate ester potassium, L-ascorbic acid sulfate ester magnesium, L-ascorbic acid sulfate ester calcium, L-ascorbic acid sulfate ester aluminum, L-ascorbic acid sodium, L-ascorbic acid potassium, L-ascorbic acid magnesium, L-ascorbic acid calcium, L-ascorbic acid aluminum and the like, and their sodium salts, potassium salts, magnesium salts, calcium salts, zinc salts, ammonium salts, alkyl-substituted ammonium salts, hydroxyalkyl-substituted ammonium salts and the like.

In addition, it may be in a form in which such an ascorbic acid derivative is bound to a polymer chain. From the viewpoint of convenience of the preparation such as solubility in water, chemical stability of the derivative and efficacy thereof, particularly ascorbic acid-2-phosphate and ascorbic acid-2-glucoside, and particularly salts thereof are preferred.

8. As a pH regulator, a storing stabilizer or an organic acid having a chelating effect and a salt thereof are preferred examples. The pH regulator is at least one kind selected from the group consisting of erythorbic acid and a salt thereof, dibutylhydroxytoluene, tocopherol and a derivative thereof, porphyrin, butylhydroxyanisole, sodium bisulfite, anhydrous sodium sulfite, gallic acid and a derivative thereof, alanine, sodium hydroxyethyl ethylenediamine triacetate, ethylenediamine tetraacetic acid and a salt thereof, citric acid and a salt thereof, gluconic acid, tartaric acid, phytic acid, sodium polyphosphate and sodium metaphosphate. The blending amount thereof may be in the range of 0.01% to 50% by weight relative to the total weight of the composition for external use, and preferably it may be added in the range of 0.1% to 5% by weight. As the salt, it is not particularly limited, however, a metal other than transition metals is desired from the viewpoint of its safety for the skin, and particularly sodium, potassium, magnesium and calcium are desired.

### 9. Oxygen radical scavenging agent ,

In order to promote the oxygen radical scavenging effect of the fullerene of the invention of this application, it is preferred that another oxygen radical scavenging agent be administered at the same time. Examples of the oxygen radical scavenging agent include superoxide dismutase, mannitol, bilirubin, cholesterol, tryptophan, histidine, quercetin, quercitrin, catechin, catechin derivatives, rutin and rutin derivatives, taurine, thiotaurine, egg shell membrane extract, gallic acid and gallic acid derivatives, yeast extract, Ganoderma lucidum extract, Yashajitsu extract, Geranium extract, moutan bark extract, Melissa extract, parsley extract, lycii cortex extract, a vitamin A group such as retinol and derivatives thereof (such as retinol palmitate and retinol acetate), retinal and derivatives thereof and dehydroretinal, a vitamin B group such as thiamines (such as thiamine hydrochloride and thiamine sulfate), riboflavins (such as riboflavin and riboflavin acetate), pyridoxines (such as pyridoxine hydrochloride and pyridoxine dioctanoate), flavin adenine nucleotide, cyanocobalamin, folic acids, nicotinic acids (such as nicotinic acid amide and nicotinic acid benzyl) and cholines, a vitamin D group such as ergocalciferol, cholecalciferol and dihydroxystanal, a vitamin E group such as tocopherol and derivatives thereof (such as dl-α (β or γ) -tocopherol, dl-α-tocopherol acetate, dl-α- tocopherol nicotinate, dl-α-tocopherol linoleate and dl-α-tocopherol succinate), a vitamin E group such as ubiquinones, dibutylhydroxytoluene, butylhydroxyanisole and the like.

### 10. Other agents to be added

For the purpose of preserving the composition for external use containing the fullerene of the invention of this application or a diluted solution thereof, an antiseptic such as ethanol can be added. In addition, a pH regulator such as an organic acid including citric acid, fumaric acid, succinic acid, lactic acid and the like or NaOH or KOH can be added to adjust the pH.

In any case, to the composition for external use containing the fullerene of the invention of this application, one or more kinds of components to be used generally for a pharmaceutical preparation such as a preparation for external use, that is, water (such as purified water, spring water, deep-sea water), an oil, a surfactant, a metal soap, a gelling agent, a powder, an alcohol, a water-soluble polymer, a film-forming agent, a resin, a ultraviolet protective agent, a clathrate compound, antimicrobial agent, a flavor, a deodorant, a salt, a pH regulator, a refreshing agent, an extract derived from an animal or a microorganism, an extract derived from a plant, a blood circulation promoter, an astringent, an antiseborrheic agent, an oxygen radical scavenging agent, a cell activator, a moisturizer, a keratolytic agent, an enzyme, a hormone, a vitamin and the like can be added as needed within the range not adversely affecting the pharmacological effect.

In addition, examples of the ultraviolet protective agent include cinnamic acid-based ultraviolet absorbents such as 2-ethylhexyl p-methoxycinnamate, isopropyl p-methoxycinnamate, p-methoxyhydrocinnamate diethanolamine salt, di-p-methoxycinnamate-mono-2-ethylhexanoate glyceryl, octyl methoxycinnamate and methyl diisopropylcinnamate, benzophenone-based ultraviolet absorbents such as 2-hydroxy-4-methoxybenzophenone, 2-hydroxy-4-methoxy-benzophenone-5-sulfuric acid, 2-hydroxy-4-methoxy-benzophenone-5-sulfate sodium, 2,4-dihydroxy-benzophenone, 2,2' -dihydroxy-4,4' -dimethoxy-benzophenone, 2,2' -dihydroxy-4-methoxy-benzophenone, 2,2' ,4,4' -tetrahydroxybenzophenone, and 2-hydroxy- 4-n-octoxybenzophenone, benzoic acid-based ultraviolet absorbents such as p-aminobenzoic acid, ethyl p-aminobenzoate, butyl p-aminobenzoate, p-dimethyl-aminobenzoate-2-ethylhexyl, glyceryl p-aminobenzoate, and amyl p-aminobenzoate, salicylic acid-based ultraviolet absorbents such as salicylate-2-ethylhexyl, salicylate triethanolamine, homomenthyl salicylate, salicylate dipropylene glycol, methyl salicylate, salicylate ethylene glycol, phenyl salicylate, amyl salicylate, benzyl salicylate, isopropylbenzyl salicylate, and potassium salicylate, dibenzoylmethane-based ultraviolet absorbents such as 4-t-butyl-4'-methoxydibenzoylmethane, 4-isopropyl-dibenzoylmethane, 4-methoxydibenzoylmethane, and 4-t-butyl-4'-hydroxydibenzoylmethane, menthyl-O-amino-benzoate, 2-phenyl-benzimidazole-5-sulfuric acid, 2-phenyl-5-methylbenzoxazole, 3-(4-methylbenzylidene)-camphor, 2-ethylhexyl-2-cyano-3,3'-diphenyl acrylate, 2-ethyl-2-cyano-3,3'-diphenyl acrylate, 2-(2' -hydroxy-5-methylphenyl)benzotriazole, anthranilic acid-based ultraviolet absorbents such as menthyl anthranilate, urocanic acid-based ultraviolet absorbents such as ethyl urocanate, titanium oxide, zirconium oxide, cerium oxide and the like. Such a metal oxide may be coated with silica. The blending amount of the ultraviolet protective agent may be 0.001 to 50% by weight, and is preferably 0.01 to 10%.

Examples of the antimicrobial agent include benzoic acid, sodium benzoate, carbolic acid, sorbic acid, potassium sorbate, p-hydroxybenzoate ester, p-chloro-m-cresol, hexachlorophene, benzalkoonium chloride, chlorhexidine chloride, trichlorocarbanilde, photosensitive elements, zinc bis(2-pyridylthio-1-oxide) phenoxyethanol, thiantol, isopropyl- methylphenol and the like. Examples of the pH regulator include potassium carbonate, sodium bicarbonate, ammonium bicarbonate and the like. Examples of the refreshing agent include L-menthol, camphor and the like.

Examples of the antiinflammatory agent include glycyrrhizinic acids or glycyrrhetinic acids such as dipotassium glycyrrhizinate, monoammonium glycyrrhizinate, β-glycyrrhetinic acid, stearyl glycyrrhetinate, disodium 3-succinyloxy glycyrrhetinate, derivatives thereof and salts thereof, mefenamic acid, phenylbutazone, indomethacin, ibuprofen, ketoprofen, allantoin, guaiazulene, panthenols such as calcium pantothenate, D-pantothenyl alcohol, pantothenyl ethyl ether, acetyl pantothenyl ethyl ether, derivatives thereof and salts thereof, ε-aminocapronic acid, diclofenac sodium, tranexamic acid, and the like. The blending amount is 0.001 to 10% by weight, and more preferably 0.01 to 5% by weight.

Examples of the antioxidant include superoxide dismutase, mannitol, histidine, tryptophan, bilirubin, quercetin, quercitrin, polyphenol, proanthocyanidin, tocotrienol, catechin, catechin derivatives, rutin and derivatives thereof, gallic acid and derivatives thereof, ubiquinone, astaxanthin, carotene, and other retinols such as retinol palmitate and retinol acetate and derivatives thereof, retinal and derivatives thereof, dehydroretinal, carotenoids such as carotene, lycopene and astaxanthin, carotenoids, a vitamin B group such as thiamine hydrochloride, thiamine sulfate, riboflavin, riboflavin acetate, pyridoxines such as pyridoxine hydrochloride and pyridoxine dioctanoate, flavin adenine nucleotide, cyanocobalamin, folic acids, nicotinic acids such as nicotinic acid amide and nicotinic acid benzyl and cholines, a vitamin D group such as ergocalciferol, cholecalciferol and dihydroxystanal, a vitamin E group such as tocopherol such as dl-α (β or γ) -tocopherol, dl-α-tocopherol acetate, dl-α-tocopherol nicotinate, dl-α-tocopherol linoleate and dl-α-tocopherol succinate and derivatives thereof, and ubiquinones, dibutylhydroxytoluene, butylhydroxyanisole and the like.

### <D> Stable external preparation containing fullerene

In the case where the composition for external use of the invention of this application is applied as an external preparation in a more practical manner, it is preferred to include at least one kind of the foregoing components, namely:
<1> a nonionic surfactant,
<2> ascorbic acid or a derivative thereof or a salt thereof, and
<3> ultraviolet protective agent.

More preferably, the nonionic surfactant in <1> is present in the range of 0.01 % by weight to 50% by weight of the total composition and is one or more kinds selected from the following members. That is, it is at least one kind selected from a POE sorbitan fatty acid ester such as POE sorbitan monooleate, POE sorbitan monostearate, POE sorbitan monolaurate or POE sorbitan tetraoleate, a POE sorbitol fatty acid ester such as POE sorbitol monolaurate, POE sorbitol monooleate, POE sorbitol pentaoleate or POE sorbitol monostearate, a POE glycerin fatty acid ester such as POE glycerin monostearate or POE glycerin triisostearate, a POE fatty acid ester such as POE monooleate, a POE alkyl ether such as POE lauryl ether, a POE alkylphenyl ether such as POE octylphenyl ether, a POE-POP alkyl ether such as POE-POP cetyl ether, a tetra POE-tetra POE ethylenediamine condensate, POE castor oil, a hardened castor oil derivative, a derivative of POE beeswax and lanolin, an alkanolamide such as lauric monoethanolamide, a POE propylene glycol fatty acid ester, a POE alkylamine, a POE fatty acid amide, a sucrose fatty acid ester, a POE nonylphenyl formaldehyde condensate, an alkyl ethoxy dimethylamine oxide, trioleyl phosphate and a polyglycerin fatty acid ester.

In addition, the ascorbic acid in <2> is present in the range of 0.01 % by weight to 20% by weight, further, in the range of 0.1 to 10% by weight relative to the total amount of the composition and is one or more kinds selected from the compound represented by the foregoing formula (1) and the salt thereof.

The ultraviolet protective agent in <3> is present in the range of 0.01 % by weight to 50% by weight relative to the total amount of the composition and is one or more kinds selected from the following members. That is, the members are a cinnamic acid-based ultraviolet absorbent such as 2-ethylhexyl p-methoxycinnamate, isopropyl p-methoxycinnamate, p-methoxyhydrocinnamate diethanolamine salt, glyceryl mono-2-ethylhexanoate di-p-methoxycinnamate, octyl p-methoxycinnamate or methyl diisopropylcinnamate, a benzophenone-based ultraviolet absorbent such as 2-hydroxy-4-methoxybenzophenone, 2-hydroxy-4-methoxy-benzophenone-5-sulfuric acid, 2-hydroxy-4-methoxy-benzophenone-5-sodium sulfate, 2,4-dihydroxy-benzophenone, 2,2'-dihydroxy-4,4'-dimethoxy-benzophenone, 2,2'-dihydroxy-4-methoxybenzophenone, 2,2',4,4'-tetrahydroxybenzophenone or 2-hydroxy-4-n-octoxybenzophenone, a benzoic acid-based ultraviolet absorbent such as p-aminobenzoic acid, ethyl p-aminobenzoate, butyl p-aminobenzoate, 2-ethylhexyl p-dimethylaminobenzoate, glyceryl p-aminobenzoate or amyl p-aminobenzoate, a salicylic acid-based ultraviolet absorbent such as 2-ethylhexyl salicylate, triethanolamine salicylate, homomenthyl salicylate, dipropylene glycol salicylate, methyl salicylate, ethylene glycol salicylate, phenyl salicylate, amyl salicylate, benzyl salycylate, isopropylbenzyl salicylate or potassium salicylate, a dibenzoylmethane-based ultraviolet absorbent such as 4-t-butyl-4' -methoxydibenzoylmethane, 4-isopropyl-dibenzoylmethane, 4-methoxydibenzoylmethane or 4-t-butyl-4'-hydroxydibenzoylmethane, menthyl-O-amino-benzoate, 2-phenyl-benzimidazole-5-sulfuric acid, 2-phenyl-5-methylbenzoxazole, 3-(4-methylbenzilidene)-camphor, 2-ethylhexyl-2-cyano-3,3-diphenylacrylate, 2-ethyl-2-cyano-3,3' -diphenyl-acrylate, 2-(2' -hydroxy-5-methylphenyl)benzotriazole, an anthranilic acid-based ultraviolet absorbent such as menthyl anthranilate, an urocanic acid-based ultraviolet absorbent such as ethyl urocanate, titanium oxide, zirconium oxide, cerium oxide, zinc oxide and a silica-coated metal oxide thereof.

In the invention of this application, in order to make the composition a stable composition, it is preferred that one or more kinds of the following members be blended in the range of 0.01% by weight to 10% by weight relative to the total of the composition as <4> a storing stabilizer or an organic acid having a chelating effect or a salt thereof. That is, it is at least one kind selected from the group consisting of erythorbic acid and a salt thereof, dibutylhydroxytoluene, tocopherol and a derivative thereof, porphyrin, butylhydroxyanisole, sodium bisulfite, anhydrous sodium sulfite, gallic acid and a derivative thereof, alanine, sodium hydroxyethyl ethylenediamine triacetate, ethylenediamine tetraacetic acid and a salt thereof, citric acid and a salt thereof, gluconic acid, tartaric acid, phytic acid, sodium polyphosphate and sodium metaphosphate. The blending amount thereof is in the range of 0.01% to 50% by weight relative to the total weight of the composition for external use, and preferably, it may be added in the range of 0.1% to 5% by weight. As the salt, it is not particularly limited, however, a metal other than transition metals is desired from the viewpoint of its safety for the skin, and particularly sodium, potassium, magnesium and calcium are desired.

In such a stabilized composition, as a water based composition, it is particularly preferred that the pH value be in the range of 3 to 10 as above. In addition, for the formulation of the stabilized composition, it is particularly desired that the total concentration of transition metal compounds be 0.1% or less.

When the antioxidative ability of a fullerene is applied to and used in the composition for external use, generally due to the potent antioxidative ability of the fullerene, the fullerene itself is susceptible to oxidization, and even if it is blended in the composition for external use, it is likely to become unstable in the pharmaceutical preparation.

In other words, due to the potent antioxidative ability of the fullerene, the fullerene exerts a reductive activity in the composition for external use and is rapidly oxidized. As a result, when it is applied to the skin, its antioxidative ability is decreased and weakened, or inactivated, whereby it cannot exert sufficient effect in some cases.

In addition, even if the fullerenes are applied as a preparation for external use, its active ingredient is oxidized and decomposed, and a part of the fullerenes is changed into free radicals on the skin by the effect of ultraviolet or the like, whereby the effect as an antioxidant is not sufficient, and improvement and therapeutic effects as a preparation for external use cannot be sufficiently exerted thereby resulting in exerting only a protective or a preventive effect in some cases.

With regard to such a concern, a favorable stability is attained for the foregoing composition.

### <E> External preparation with an enhanced effect

Furthermore, in the invention of this application, as a composition for a favorable external preparation in a more practical manner, by including at least one kind of the following components, the fullerene composition for external use with an enhanced effect on whitening the skin, improving pigmentation, treating acnes, improving wrinkles, improving skin roughness, improving oily skin, improving dry skin, diminishing the appearance of pores, treating scarring, treating flushing of the face, treating hair loss, promoting hair growth, treating a burn injury, sterilizing the skin, killing mites on the skin or improving skin texture is provided.

That is,
<5> a whitening component present at 0.01% by weight to 20% by weight relative to the total amount of the composition, which is at least one kind selected from cysteine, a derivative thereof and a salt thereof, glabridin, glabrene, liquiritin, isoliquiritin, placenta extract, hydroquinone and a derivative thereof, resorcinol and a derivative thereof and glutathione,
<6> an antiinflammatory component present in the range of 0.001 % by weight to 10% by weight relative to the total of the composition, which is at least one kind selected from glycyrrhizinic acid, glycyrrhetinic acid, a derivative thereof and a salt thereof, mefenamic acid, phenylbutazone, indomethacin, ibuprofen, ketoprofen, allantoin, guaiazulene, panthenol, a derivative thereof and a salt thereof, ε-aminocapronic acid, diclofenac sodium and tranexamic acid, and
<7> an antioxidative component present in the range of 0.001 % by weight to 10% by weight relative to the total of the composition, which is at least one kind selected from superoxide dismutase, mannitol, histidine, tryptophan, bilirubin, quercetin, quercitrin, polyphenol, proanthocyanidin, tocotrienol, catechin, a catechin derivative, rutin and a derivative thereof, gallic acid and a derivative thereof, ubiquinone, astaxanthin, carotene, other carotenoids, a derivative thereof and a salt thereof, a vitamin B group, a derivative thereof and a salt thereof, a vitamin D group, a derivative thereof and a salt thereof, a vitamin E group, a derivative thereof and a salt thereof, dibutylhydroxytoluene and butylhydroxyanisole.

Therefore, the invention of this application will be explained in more detail by showing Examples hereunder. It is a matter of course that the invention is not limited to the following Examples.

### Examples

### <A> Activity test of active ingredient (Part 1)

The following samples (A) to (G) were prepared.
F1: MIX fullerene containing C60 fullerene and C70 fullerene (average molecular weight 744/powder)
F2: C60 fullerene (molecular weight 720/powder)
F4: Fullerene clathrated in PVP (average molecular weight: 40,000/70 mg/ml (pure aqueous solution), PVP: molecular weight 40,000, MIX fullerene: 744, concentration of MIX fullerene: 0.3 mg/ml)
F6: Hydroxylated fullerene: average molecular weight 958.7/powder (water-soluble solid), hydroxylation ratio: 14 OH group/molecule
F7: MIX fullerene/isostearic acid, average molecular weight 744/5 mg/g (isostearic acid of solvent: molecular weight 284)
(1) With regard to the foregoing samples, according to the direction in the following Table 1, the sample was put into a 1.5 ml Eppendorf tube, H₂O₂ was added, and reaction was carried out for 30 seconds after the addition for H₂O₂. Subsequently, the measurement was carried out by ESR, and the hydroxy radical scavenging activities of the fullerenes measured by the ESR spin trapping method were evaluated. In the measurement, when DMPO, as the spin trapping agent stabilizing unstable hydroxyl radicals as the adduct, was added, the electron spin resonance spectra showing a characteristic quartet of 1:2:2:1 was observed with an electron spin resonance apparatus (JES-FR30 manufactured by JEOL Co.), therefore, the amount of hydroxyl radicals was calculated from the ratio of this intensity to the intensity of manganese oxide showing paramagnetic spectra, which was added as the relative internal standard.

**Table 1**

| Reagent | Concentration | Collection amount |
|---|---|---|
| Test reagent | - | 60 µl |
| H₂O₂ | 100 µM | 10 µl |
| FeSO₄ | 10 µM | 10 µl |
| DMPO | 1.79 M | 20 µl |

| | | |
|---|---|---|
| 5,5-Dimethyl-1-pyrroline-N-oxide (DMPO); LABOTEC Co. | | |

Ascorbic Acid (Asc); SIGMA Co.

The detail description of the test reagent is written in F-ESR-2-2. For preparation of the reagents, milli Q was used.

As for the ESR apparatus, JES-FR30 (JEOL Co.) was used, and measurement was carried out using a high sensitive ESR water-soluble flat cell (LABOTEC Co.).

The measurement conditions of ESR were as follows.

Power: 4 mW, Field: 336 ± 5, Sweep Time: 1 min., Modulation Width: 0.32 mT, Amp: 400, Responce Time: 0.3 sec.

Evaluation was performed with the ratio of the signal amplitude of OH to that of the paramagnetic internal standard, MnO.

Incidentally, the samples F6 and 7 were used after diluting the maximum amount that can be solubilized in 70% ethanol to 0.2%. In addition, DMSO is a scavenging agent of hydroxy radicals, and it has been confirmed that also ethanol has a hydroxy radical scavenging activity.

When the test results are evaluated as the remaining amount of the hydroxyl radicals: the relative signal intensity (DMPO-OH/MnO), they can be represented as in the following Table 2.

**Table 2**

| Sample | Remaining amount of hydroxyl radicals |
|---|---|
| Control | 3.0 |
| (Milli-Q water) | |
| DMSO | 0.8 |
| (12.8 M) | |
| Aₛₑ | 0.42 |
| (100 µM) | |
| A2P | 0.45 |
| (100 µM) | |
| Ethanol | 1.3 |
| F4 | 2.0 |
| (400 µM) | |
| F6 | 0.6 |
| (9.26 µM) | |
| F7 | 0.3 |
| (18.12 µM) | |

For example, as shown in this Table 2, significant hydroxyl radical scavenging activities were confirmed in two types of the fullerenes (F6 and F7) and the activity of F4 was slightly superior to or equal to that of an existing pro-vitamin C (A2P: ascorbic acid-2-phosphate Na), which is frequently used in a commercially available cosmetic product.

(2) In addition, according to the direction in the following Table 3, each of the samples was put into a 1.5 ml Eppendorf tube, XOD was added, and reaction was carried out for 30 seconds. Subsequently, the measurement was carried out by ESR, whereby the superoxide scavenging activities measured by ESR spin trapping were evaluated. The measuring method was according to the case of the hydroxyl radical. When a spin trapping agent, DMPO, was added, the electron spin resonance spectra showing a slightly weak quartet of 1:1:1:1 was observed with an electron spin resonance apparatus (JES-FR30 manufactured by JEOL Co.), therefore, the amount of superoxide anion radicals was calculated from the ratio of this intensity to the intensity of manganese oxide showing paramagnetic spectra, which was added as the relative internal standard.

**Table 3**

| Reagent | Concentration | Collection amount |
|---|---|---|
| Test reagent | - | 50 µl |
| HPX | 2.7 mg/5 ml | 50 µl |
| DMPO | 2.99 M | 20 µl |
| DMSO | 1.0 M | 30 µl |
| XOD | 2 µ/ml | 50 µl |

Hypoxanthine (HPX): SIGMA Co., Xanthine Oxidase (XOD): Roche Co. 5,5-Dimethyl-1-pyrroline N-oxide (DMPO); LABOTEC Co.

Ascorbic Acid (Asc); SIGMA Co.

The detail description of the test reagent is written in F-ESR-1-2. DMPO and the test reagents were prepared with milli Q. Other reagents were prepared with PBS (Dulbecco's PBS(-)):NISSUI PHARMACEUTICAL Co.

AS for the ESR apparatus, JES-FR30 (JEOL Co.) was used, and measurement was carried out using a high sensitive ESR water-soluble flat cell (LABOTEC Co.).

The measurement conditions of ESR were as follows.

Power: 4 mW, Field: 336 ± 5, Sweep Time: 1 min., Modulation Width: 0.1 mT, Amp: 790, Responce Time: 0.3 sec.

Evaluation was performed with the ratio of the signal amplitude of O₂ to that of the paramagnetic internal standard, MnO.

Incidentally, the samples F6 and 7 were used after diluting the maximum amount that can be solubilized in 70% ethanol to 0.2% in the same manner as above.

When the test results are evaluated as the remaining amount of the superoxide anion radicals: the relative signal intensity (DMPO-OOH/MnO), they can be represented as in the following Table 4.

**Table 4**

| Sample | Remaining amount of superoxide anion radical |
|---|---|
| Control | 3.0 |
| (Milli-Q water) | |
| Aₛₑ | 2.1 |
| (100 µM) | |
| A2P | 5.1 |
| (100 µM) | |
| Ethanol | 7.5 |
| F4 | 1.6 |
| (200 µM) | |
| F6 | 3.1 |
| (9.26 µM) | |
| F7 | 6.2 |
| (18.12 µM) | |

For example, as shown in this Table 4, significant hydroxyl radical scavenging activities was confirmed in F4, and the activity is significantly superior to that of an existing pro-vitamin C (A2P), which is frequently used in a commercially available cosmetic product. In addition, F6 had the scavenging activity equal to or more than that of the pro-vitamin C.

(3) With regard to various samples, their antioxidative effects against UVB were evaluated by using keratinocytes, a human major cell type. In the test method, CDCFH-DA, as a redox indicator, has been loaded into a cell, and when it is oxidized by the peroxide/ hydrogen peroxide generated by ultraviolet irradiation, fluorescence is generated. Therefore, the intensity of the fluorescence is measured with a fluorescence plate reader (type 2350 manufactured by Millipore/ Perceptive Co.).

When the skin is irradiated with sunlight, peroxide/ hydrogen peroxide is generated in cells by UVB rays in sunlight, or cell death is caused by being subjected to DNA breakage, DNA damage or cell membrane breakage.

It has been confirmed that when UVB rays are irradiated to the human skin epidermal keratinocytes at the dose of 40 mJ/cm². which triggers skin erythema formation, the amount of peroxide/hydrogen peroxide in the cells reaches the maximum value 4 minutes after the irradiation. Therefore, the samples were added in advance before the irradiation, and then the antioxidative effects were evaluated.

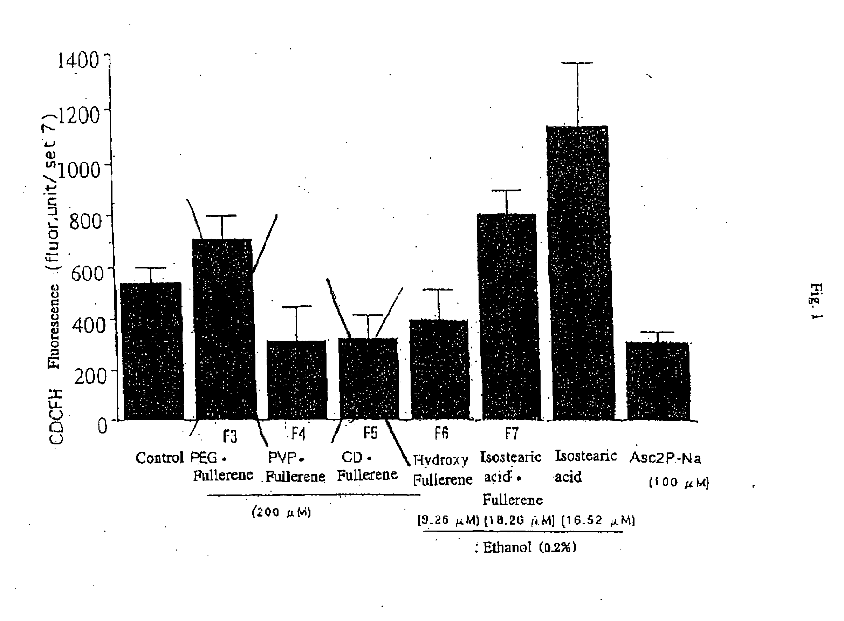

The results are shown in Fig. 1. The peroxide/hydrogen peroxide scavenging activities in the skin cells, namely, anti oxidative effects equal to that of an existing pro-vitamin C (Asc2P-Na) was confirmed in F4 and F6.

(4) With regard to the respective samples, the scavenging activities against the peroxide/hydrogen peroxide in the skin cells induced by lipid peroxide (deteriorated oil) were evaluated.

Lipids present in the skin are susceptible to oxidization at any time, whereby it becomes a cause of leading to the death of skin cells. In particular, ceramide or squalene, which is a lipid in the stratum corneum, is subjected to oxidization and changed into a hydroperoxide to cause cell death.

It has been confirmed by the inventor that when one of the hydroperoxides, t-BuOOH (tert-butylhydro peroxide) is added to the human skin epidermal keratinocytes at a concentration of 140 to 250 µM, which causes 40 to 70% cell death, the amount of peroxide/hydrogen peroxide in the cells reaches the maximum value 150 minutes after the addition. Therefore, the samples were added in advance before the addition, and then the peroxide/hydrogen peroxide scavenging activities were evaluated.

In the measuring method, CDCFH-DA, as a redox indicator, has been loaded into a cell, and when it is oxidized by the peroxide/hydrogen peroxide generated by the addition of t-BuOOH, fluorescence is generated. Therefore, the intensity of the fluorescence is measured with a fluorescence plate reader (type 2350 manufactured by Millipore/Perceptive Co.).

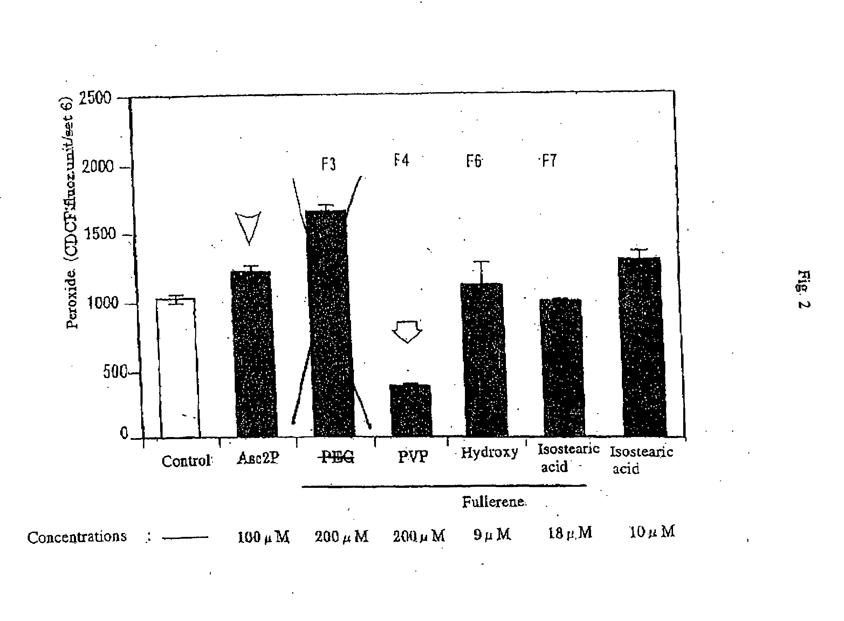

The results are shown in Fig. 2.

When t-BuOOH of a model substance of hydrocarbon peroxide, which has a cell-killing ability, was added to the human skin keratinocytes, peroxide/ hydrogen peroxide is generated in the cells, however, the scavenging activity superior to that of an existing pro-vitamin C was confirmed in one type (F4) of the fullerenes. This fullerene is the test sample in which the protective activity against ultraviolet or superoxide anion radical was also confirmed.

### <B> Activity test of active ingredient (Part 2)

First, a fullerene modified and conjugated with PVP (polyvinyl pyrrolidone) was prepared according to a known literature.

**Table 5**

| | | |
|---|---|---|
| PVP with molecular weight 60,000 in chloroform solution | | |
| ↓ | + fullerene in toluene solution | |
| Mixing | | |
| ↓ | Evaporation (exsiccation) | |
| ↓ | + H₂O | |
| Suspension | | |
| ↓ | reduced pressure (to remove toluene by azeotropy) | 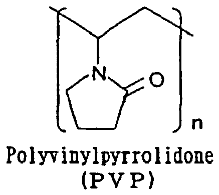 |
| Aqueous solution | | |
| ↓ | filtration | |
| Aqueous solution (product) | | |
| ↓ | | |
| Powder product (product) | | |

Here, the notation of PVP with molecular weight of 8000 indicates the one with weight-average molecular weight (determined by viscosimetry) of 60,000. More specifically, for example, first, a stirring solution in which 0.8 mg of mixed fullerenes (C60:C70 = 3.5:1 mol/mol) was dissolved in 1.0 ml of toluene was added to a chloroform solution containing 100 mg of PVP with molecular weight of 60000 at room temperature. After the solution was mixed well, the solvent was evaporated under reduced pressure. The residue was dissolved in 2.0 ml of Milli-Q water, and the suspension was subjected to azeotropic distillation to remove toluene.

Thus, an aqueous solution product was obtained. The product was further subjected to filtration and dried under reduced pressure, whereby a solid (powder) was obtained.

In the same manner, a fullerene modified and conjugated with PVP with molecular weight of 8000 whose weight-average molecular weight is 8000 was prepared.

(1) The effects in the case where the PVP-fullerenes prepared according to the foregoing method were administered into the human skin oxidized cells (HaCaT) were evaluated by measuring the cell survival rates by the WST-1 method. The WST-1 method is the method using WST-1 reagent (tetrazolium salt) generating water-soluble formazan, which develops a color by the reduction by mitochondrial dehydrogenase in the living cells, and measuring the yellow formazan generated by the reduction by mitochondrial dehydrogenase with an absorption plate reader at 450 nm.

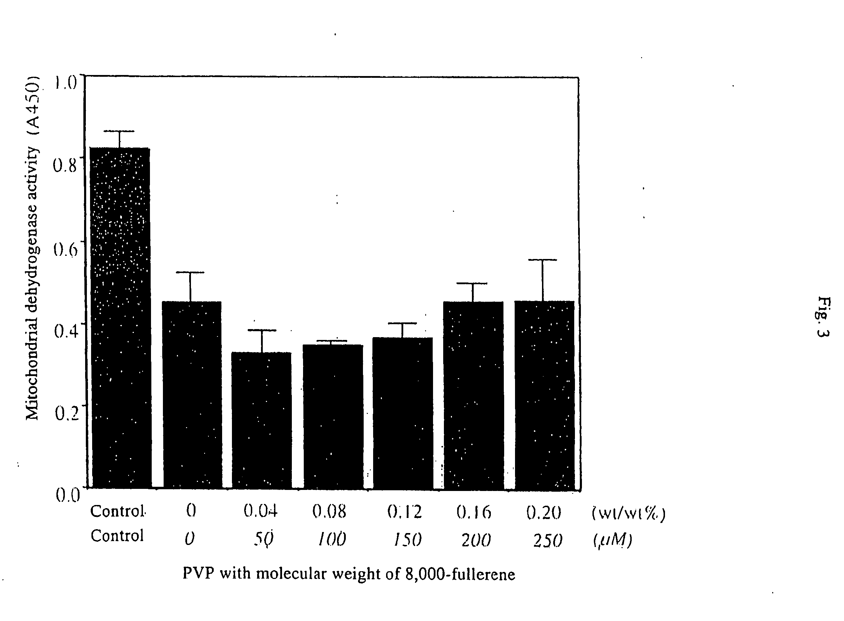

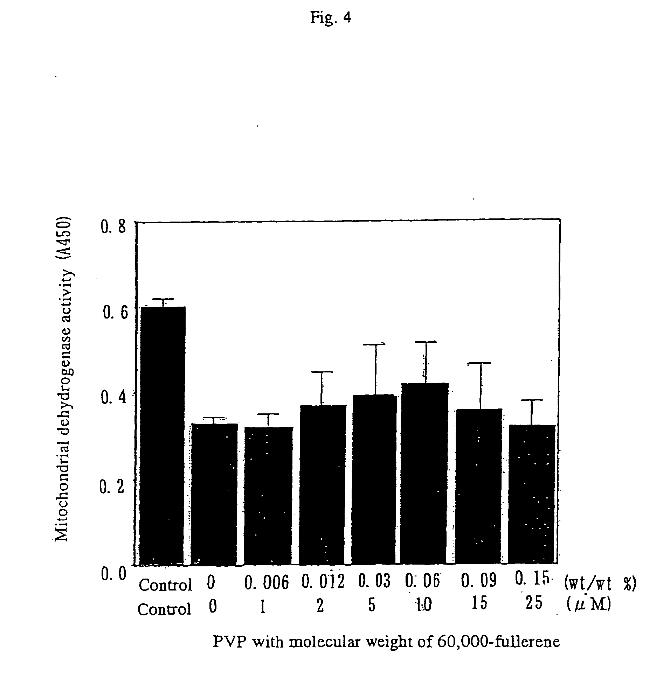

The attached Fig. 3 shows the results in the case where an aqueous solution of the fullerene modified with PVP with molecular weight of 8000 was administered in advance 3 hours before adding t-BuOOH. Fig. 4 shows the results in the case of the fullerene modified with PVP with molecular weight of 60000.

For example, from such a verification of the cell survival rates, it was confirmed that an optimal concentration of administration having the protective effect on cell death is, in the case of PVP with molecular weight of 8000 (molecular weight: 8,000), 100 to 250 µM (0.08 to 0.12 wt/wt%), in which the concentration of the fullerene is 4 to 6 ppm, and in the case of PVP with molecular weight of 60000 (molecular weight: 60,000), 10 to 25 µM (0.06 to 0.15 wt/wt%), in which the concentration of the fullerene is 3 to 7.5 ppm.

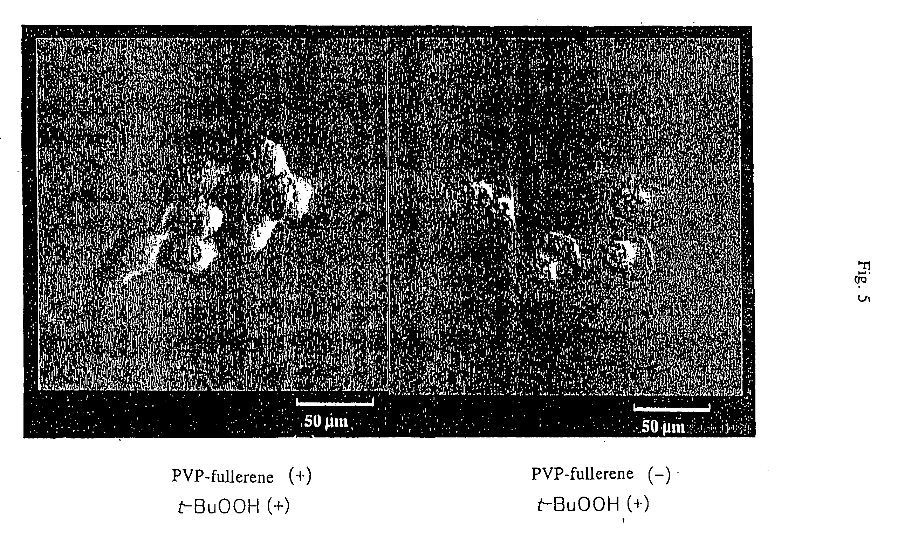

Fig. 5 shows photographs showing the protective effect of PVP-fullerene on the change in the cells of the human skin keratinocyte cells (HaCaT) caused by t-BuOOH of a model substance of lipid peroxide in the case of PVP with molecular weight of 60000. The significant protective effect was confirmed.

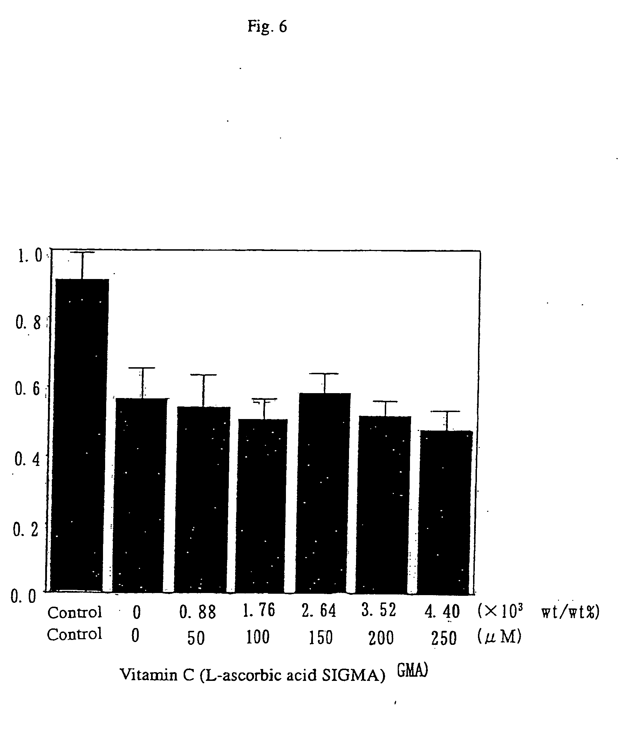

Fig. 6 shows the results of the measurements in the case of vitamin C (L-ascorbic acid) for comparison. When compared with such a vitamin C, it is confirmed that, in the case of, for example, the fullerene conjugated with PVP with molecular weight of 60000, it attains the protective effect on cell death about 10 times higher than that of vitamin C.

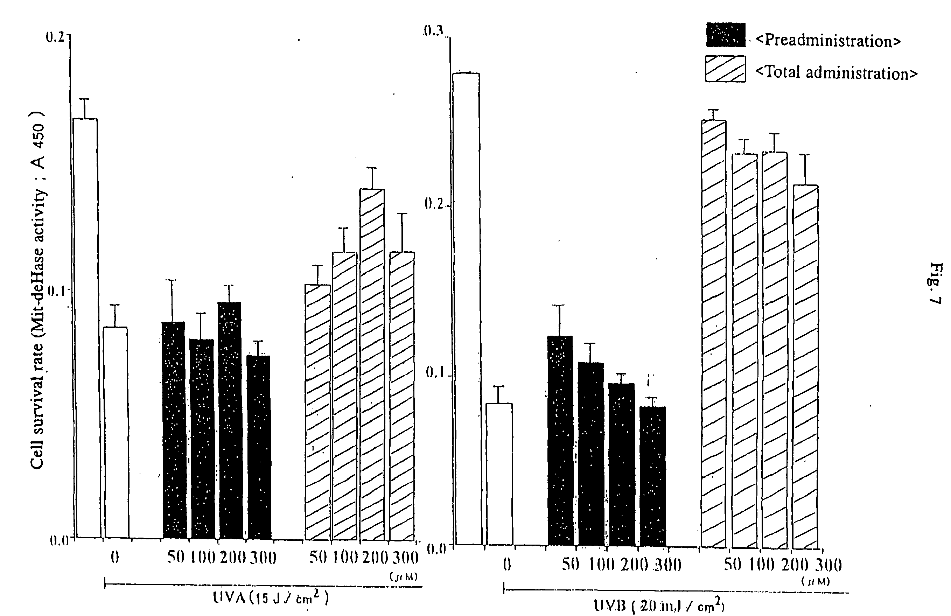

(2) Fig. 7 shows the results of evaluating the suppressing effect on cell death (cell survival rate) at UV light irradiation in the case of preadministration and total administration when PVP with molecular weight of 60000 was used.

Here, preadministration indicates the condition in which the fullerene conjugate was administered before UV irradiation and it was removed immediately before the irradiation, and total administration indicates the condition in which the fullerene conjugate was not removed during the measurement. The measurement was carried out by the WST-1 method in the same manner as in the foregoing (1).

It is demonstrated that significant effects were achieved in the case of the total administration of the PVP-fullerene conjugate, and with regard to UVB irradiation, significant effects were achieved also in the case of the preadministration.

### <C> Composition for external use and the effect thereof

### 1) Formulation of composition

By using the fullerenes in the foregoing <A>, the following compositions were prepared.

**Lotion 1 of the invention:**

| | | |
|---|---|---|
| (Content of total transition metal: 54 ppm) | | |
| F1 | | 0.1% |
| Citric acid | | 1.0% |
| L-ascorbic acid-2-phosphate sodium | | 1% |
| POE sorbitan monooleate | | 0.5% |
| 2-ethylhcxyl p-methoxycinnamate | | 0.1% |
| Xanthan gum | | 0.1% |
| NaOH (to adjust the pH to 8) | | |
| Purified water | | balance |
| Lotion 3 of the invention: | | |
| (Content of total transition metal: 60 ppm) | | |
| F4 | | 0.1% |
| L-ascorbic acid-2-phosphate sodium | | 0.5% |
| Xanthan gum | | 0.1% |
| NaOH (to adjust the pH to 8) | | |
| Purified water | | balance |
| | | |
| Lotion 5 of the invention: | | |
| (Content of total transition metal; 35 ppm) | | |
| F6 | | 0.1% |
| 2-ethylhexyl p-methoxycinnamate | | 0.1% |
| Xanthan gum | | 0.1% |
| NaOH (to adjust the pH to 8) | | |
| Purified water | | balance |
| | | |
| Control lotion 1: | | |
| F1 | | 0.1% |
| Citric acid | | 1.0% |
| L-ascorbic acid-2-phosphate sodium | | 1% |
| POE sorbitan monooleate | | 0.5% |
| 2-ethylhexyl p-methoxycinnamate | | 0.1% |
| Xanthan gum | | 0.1% |
| NaOH (to adjust the pH to 8) | | |
| Purified water | | balance |
| Zinc oxide | | 0.1% |
| Ferric oxide | | 0.1% |
| | | |
| Control lotion 2: | | |
| F1 | | 0.1% |
| Citric acid | | 1.0% |
| L-ascorbic acid-2-phosphate sodium | | 1% |
| POE sorbitan monooleate | | 0.5% |
| 2-cthylhexyl p-methoxycinnamate | | 0.1% |
| Xanthan gum | | 0.1% |
| NaOH (to adjust the pH to 10.5) | | |
| Purified water | | balance |
| | | |
| Control lotion 3: | | |
| F1 | | 0.1% |
| Xanthan gum | | 0.1% |
| Purified water | | balance |

Face lotion 1 of the invention:
(Formulation) (Hereinafter the amount is shown by % by weight.)
   (1) F1: 0.5, (2) 1,3-butylene glycol: 5.5, (3) polyoxyethylene (20 E.O.) sorbitan 1.2 monolaurate ester: 5.0, (4) ethyl alcohol: 8.0, (5) oolong tea extract (*1): 1.0, (6) seaweed extract (*2): 1.0, (7) L-ascorbic acid phosphate magnesium (*3): 0.5, (8) 2-hydroxy-4- methoxybenzophenone-5-sodium sulfate (*4): 1,0, (9) ε-aminocapronic acid (*5): 0.2, (10) antiseptic: q.s., (11) flavor: q.s., (12) purified water: balance (The pH was adjusted to 7 ± 1 with 1% citric acid and NaOH)
   *1: manufactured by Maruzen Pharmaceutical Co., *2; manufactured by Ichimaru Pharcos Co., *3: manufactured by Wako Pure Chemical Industries, Ltd., *4: manufactured by Merck Ltd., *5: manufactured by Sigma Co. (The pH was adjusted to 7 ± 1 with 1% citric acid and NaOH.)
   (Content of total transition metals: 89 ppm)
Face lotion 2 of the invention:
   (Formulation) (Hereinafter the amount is shown by % by weight.)
   (1) F2: 0.5, (2) 1,3-butylene glycol: 6.5, (3) polyoxyethylene (20 E.O.) sorbitan 1.2 monolaurate ester: 3.5, (4) ethyl alcohol: 8.0, (5) oolong tea extract (*1): 1.0, (6) seaweed extract (*2): 1.0, (7) L-ascorbic acid phosphate magnesium (*3): 0.5, (8) 2-hydroxy-4- methoxybenzophenone-5-sodium sulfate (*4): 1.0, (9) ε-aminocapronic acid (*5); 0.2, (10) antiseptic: q.s., (11) flavor: q.s., (12) purified water: balance (The pH was adjusted to 7 ± 1 with 1% citric acid and NaOH.)
   *1: manufactured by Maruzen Pharmaceutical Co., *2: manufactured by Ichimaru Pharcos Co., *3: manufactured by Wako Pure Chemical Industries, Ltd., *4: manufactured by Merck Ltd., *5: manufactured by Sigma Co.
   (Content of total transition metals: 95 ppm)
Emulsion of the invention:
   (Formulation) (%)
   (1) polyoxyethylene (10 E.O.) sorbitan 1.0 monolaurate: 7.0, (2) polyoxyethylene (60 E.O.) sorbitol 0.5 tetraoleate; 2.0, (3) glyceryl monostearate: 1.0, (4) stearic acid. 0.5, (5) behenyl alcohol: 0.5, (6) F4: 6.0, (7) jojoba oil (*1): 1.0, (8) L-ascorbic acid tetraisopalmitate (*2): 2.0, (9) barley extract (*3): 0.1, (10) carrot extract (*4): 0.1, (11) D-panthenol (*5): 0.1, (12) retinol palmitate (*6): 0.01, (13) antiseptic: 0.1, (14) carboxy vinyl polymer: 0.1, (15) sodium hydroxide: 0.05, (16) ethyl alcohol: 5.0, (17) purified water: balance, (18) flavor: q.s. (The pH was adjusted to 7 ± 1 with 1% citric acid and NaOH.)
   *1: manufactured by Kokyu Alcohol Kogyo Co., *2: manufactured by Nihon Surfactant Kogyo KK., *3: manufactured by Technoble Co., *4: manufactured by Maruzen Pharmaceutical Co., *5: manufactured by Sigma Co. *6: manufactured by Nippon Roche KK.
   (Content of total transition metals: 93 ppm)
Cream of the invention:
   (Formulation) (%)
   (1) polyoxyethylene (40 E.O.) monostearate: 2.0 (2) glycerin monostearate (self-emulsifying type): 5.0, (3) stearic acid: 5.0, (4) behenyl alcohol (*1): 0.5, (5) squalene: 15.0, (6) cetyl isooctanoate: 5.0, (7) 1,3-butylene glycol: 5.0, (8) F7: 1.0, (9) white birch extract (*2): 0.1, (10) Saxifraga stolonifera extract (*3): 0.2, (11) L-ascorbic acid-2-phosphate sodium (*4): 1.0, (12) 2-ethylhexyl p-mcthoxycinnamate (*5): 5.0, (13) riboflavin (*6): 0.05, (14) cysteine (*7): 0.1, (15) purified water: balance, (16) antiseptic: q.s., (17) flavor: q.s. (The pH was adjusted to 7 ± 1 with 1% citric acid and NaOH.)
   *1: manufactured by Sciwakasei Co., *2: manufactured by Maruzen Pharmaceutical Co., *3: manufactured by Ichimaru Pharcos Co., *4: manufactured by Sigma Co., *5: manufactured by BASF Co. *6: manufactured by Sigma Co., *7: Cycteine (manufactured by Wako Pure Chemical Industries, Ltd.) was used after diluting it to 1.0 mg/ml.
   (Content of total transition metals: 85 ppm)
Sunscreen emulsion of the invention:
   (Formulation) (%)
   (1) stearic acid: 2.0, (2) cetanol: 1.0, (3) polyoxyethylene sorbitan monooleate (20 E.O.): 0.5, (4) sorbitan sesquioleate: 0.5, (5) 2-ethylhexyl p-methoxy- cinnamate (*1): 8.0, (6) cetyl 2-ethylhexanoate: 12.0, (7) 1,3-butylene glycol: 10.0, (8) carboxy vinyl polymer: 0.2, (9) triethanolamine: 0.5, (10) peppermint extract (*2): 0.02, (11) F4: 0.1, (12) Glycyrrhiza extract (*3): 0.02, (13) placenta extract (*4): 0.3, (14) dl-α-tocopherol acetate (*5): 0.2, (15) purified water: balance, (16) antiseptic: q.s., (17) titanium oxide: 3.0, (18) flavor: q.s. (The pH was adjusted to 7 ± 1 with 1% citric acid and NaOH.)
   *1: manufactured by Maruzen Pharmaceutical Co., *2: manufactured by Ichimaru Pharcos Co., *3: manufactured by Maruzen Pharmaceutical Co., *4: manufactured by Nichirei Co., *5: manufactured by Eisai Co.
   (Content of total transition metals: 78 ppm)
Liquid foundation of the invention:
   (Formulation) (%)
   (1) lanolin: 7.0, (2) liquid paraffin: 5.0, (3) stearic acid: 2.0, (4) cetanol: 1.0, (5) macadamia nut oil (*1): 5.0, (6) F6: 0.5, (7) Cydonia oblonga extract (*2): 0.5, (8) glycerin: 5.0, (9) triethanolamine: 1.0, (10) carboxy methyl cellulose: 0.7, (11) purified water: balance, (12) mica: 15.0, (13) talc: 6.0, (14) titanium oxide: 3.0, (15) coloring pigment: 6.0, (16) L-ascorbic acid dipalmitate (*3): 0.1, (17) D-panthenol (*4): 0.01, (18) 4-t-butyl-4'-methoxydibenzoylmethane (*5): 3.5, (19) glutathione (*6): 0.005, (20) antiseptic: q.s., (21) flavor: q.s. (The pH was adjusted to 7 ± 1 with 1% citric acid and NaOH. The content of total transition metals was 100 ppm or less.)
   * 1: manufactured by Nihon Surfactant Kogyo KK., *2: manufactured by Koei Kogyo Co., *3: manufactured by Nihon Surfactant Kogyo KK., *4: manufactured by Nippon Roche KK., *5: manufactured by Givaudan Co. *6: manufactured by Sigma Co.
Facial mask of the invention:
   (Formulation) (%)
   (1) polyvinyl alcohol: 20.0, (2) ethyl alcohol: 20.0, (3) glycerin: 5.0, (4) kaolin: 6.0, (5) Malva sylvestris extract (*1): 0.05, (6) F2: 0.5, (7) Lily extract (*2): 0.05, (8) resorcin (*3): 0.02, (9) riboflavin (*4): 0.1, (10) trancxamic acid (*5): 0.5, (11) antiseptic: 0.2, (12) flavor: 0.1, (13) purified water: balance (The pH was adjusted to 7 ± 1 with 1% citric acid and NaOH. The content of total transition metals was 100 ppm or less.)
   *1: manufactured by Maruzen Pharmaceutical Co., *2: manufactured by Gatehose Co., *3: manufactured by Sigma Co., *4: manufactured by Sigma Co., *5: manufactured by Sigma Co.
Cleansing agent of the invention:
   (Formulation) (%)
   (1) stearic acid: 10.0, (2) palmitic acid: 8.0, (3) myristic acid: 12.0, (4) lauric acid: 4.0, (5) oleyl alcohol: 1.5, (6) purified lanolin: 1.0, (7) astaxanthin (*1): 0.005, (8) flavor: 0.1, (9) antiseptic: 0.2, (10) glycerin: 18.0, (11) potassium hydroxide: 6.0, (12) F1: 0.5, (13) Saponaria officinalis extract (*2): 0.5, (14) dipotassium glycyrrhizinate (*3): 0.2, (15) L-ascorbic acid palmitate (*4): 0.05, (16) purified water: balance (The pH was adjusted to 7 ± 1 with 1% citric acid and NaOH. The content of total transition metals was 100 ppm or less.)
   *1: manufactured by Sigma Co., *2: manufactured by lchimaru Pharcos Co., *3: manufactured by Maruzen Pharmaceutical Co., *4: manufactured by Sigma Co.

### 2) Stability test

A stability test was carried out at 45°C for 3 months by using the foregoing Lotions 1 to 5 of the invention and Control lotions 1 to 3. The hydroxy radical scavenging activities of the lotions before and after the stability test were measured by the ESR trapping method after adding hydrogen peroxide, and the results of showing the remaining activities as percentage are shown below.
(Remaining ratio of hydroxy radical scavenging activity)

| | |
|---|---|
| Lotion 1 of the invention | 91% |
| Lotion 3 of the invention | 94% |
| Lotion 5 of the invention | 82% |
| Control lotion 1 | 12% |
| Control lotion 2 | 45% |
| Control lotion 3 | 59% |

From the above results, it has been confirmed that high activities of the fullerene compositions for cosmetic skin treatment use of this invention are maintained.

In addition, formulations in which all of the storing stabilizer, the nonionic surfactant, the ascorbic acid derivative, the ultraviolet protective agent were removed from each of the above formulations of the invention of this application, were prepared as a control group, and a stability test was carried out at 40°C for I month.

Evaluation of the stability test was carried out by evaluating change in the color of the formulation, occurrence of precipitation and occurrence of odor by grading them as the following scores, obtaining the average scores and comparing them. With regard to the evaluation, 10 males and 10 females aged between 20 and 50 years evaluated the formulations, respectively and the average scores were obtained.

### (Score)

Change in color: change is not observed at all (score 0), change is observed (score 2), significant change is observed (score 3)
Change in precipitation: change is not observed at all (score 0), precipitation is observed (score 2), significant precipitation is observed (score 3)
Occurrence of odor: change is not observed at all (score 0), change is observed (score 2), significant change is observed (score 3)

As a result, any changes over time with regard to all the items, namely, change in color, occurrence of precipitation and change in odor were not observed for the formulations of the invention of this application, whereby the score was 0, however, for the formulated cosmetic products in the control group, in which all of the storing stabilizer, the nonionic surfactant, the ascorbic acid derivative, the ultraviolet protective agent were removed, the average score was not 0 but from 2 to 3. From this result, it has been demonstrated that the formulations of this invention have an excellent stability.

### 3) Effect test

Among the patients visiting the cosmetic dermatology department, 20 male patients and 20 female patients from aged between 15 and 75 years, who asked for whitening the skin, improving pigmentation, treating acnes, improving wrinkles, improving skin roughness, improving oily skin, improving dry skin, diminishing the appearance of pores, treating scarring, treating flushing of the face, treating hair loss, promoting hair growth, treating a burn injury, sterilizing the skin, killing mites on the skin or improving skin texture were selected for each group, respectively. The patients were divided into two groups, one of which was a group using the preparation of Lotion 6 of the invention of the following formulation, and the other was a placebo group. The preparations were applied to the affected part twice a day in the morning and at night during the period between 59 days and 97 days, and the results of the questionnaire survey were examined based on the scores. The results are summarized in Table 2.

### (Evaluation criteria)

### [Score] [Condition]

0: significant effect is observed, 1: effect is observed, 2: very slight effect is observed,
3: effect is not observed

According to the above evaluation criteria, the number of the patients who graded the formulation as a score of 1 or lower out of 20 patients was counted, respectively, and it was judged according to the following judgment criteria.

### (Judgment criteria)

[Judgment] [Content] Very effective: The number of patients out of 20 who graded the formulation as a score of 1 or lower is 16 or more.

Effective: The number of patients out of 20 who graded the formulation as a score of 1 or lower is 12 to 14.

Slightly effective: The number of patients out of 20 who graded the formulation as a score of 1 or lower is 8 to 10.

Ineffective: The number of patients out of 20 who graded the formulation as a score of 1 or lower is 6 or lower.

**Lotion 6 of the invention:**

| | |
|---|---|
| F4 | 0.1% |
| Citric acid | 1.0% |
| L-ascorbic acid-2-phosphate sodium | 0.8% |
| POE sorbitan monooleate | 0.5% |
| 2-ethylhexyl p-methoxycinnamate | 0.2% |
| Xanthan gum | 0.1% |
| Glabridin | |
| Glycyrrhizinic acid | |
| Nicotinic acid amide | |
| NaOH (to adjust the pH to 8) | |
| Purified water | balance |
| | |
| Placebo lotion: | |
| Citric acid | 1.0% |
| L-ascorbic acid-2-phosphate sodium | 1% |
| POE sorbitan monooleate | 0.5% |
| 2-ethylhexyl p-methoxycinnamate | 0.1% |
| Xanthan gum | 0.1% |
| Glabridin | |
| Glycyrrhizinic acid | |
| Nicotinic acid amide | |
| NaOH (to adjust the pH to 8) | |
| Purified water balance | |

**Table 6 (Results of the effects on each disease)**

| | Lotion of the invention | Placebo |
|---|---|---|
| Whitening the skin | Very effective | Slightly effective |
| Improving pigmentation | Very effective | Slightly effective |
| Treating acnes | Very effective | Slightly effective |
| Improving wrinkles | Very effective | Ineffective |
| Improving skin roughness | Very effective | Ineffective |
| Improving oily skin | Very effective | Slightly effective |
| Improving dry skin | Very effective | Ineffective |
| Diminishing the appearance of pores | Very effective | Slightly effective |
| Treating scarring | Very effective | Slightly effective |
| Treating flushing of the face | Very effective | Ineffective |
| Treating hair loss | Very effective | Ineffective |
| Promoting hair growth | Very effective | Ineffective |
| Treating a burn injury | Very effective | Slightly effective |
| Improving skin texture | Very effective | Ineffective |
| Sterilizing the skin | Very effective | Ineffective |
| Killing mites on the skin | Very effective | Ineffective |

From the results, the excellent effect of the fullerene-containing composition for external use of the invention of this application on all of the target diseases, namely, whitening the skin, improving pigmentation, treating acnes, improving wrinkles, improving skin roughness, improving oily skin, improving dry skin, diminishing the appearance of pores, treating scarring, treating flushing of the face, treating hair loss, promoting hair growth, treating a burn injury, sterilizing the skin, killing mites on the skin and improving skin texture has been confirmed.

### Industrial Applicability

According to the invention of this application, it has excellent advantages that, by including at least one kind of a storing stabilizer, a nonionic surfactant, an ascorbic acid derivative and an ultraviolet protective agent, the stability can be improved, and moreover, by including at least one kind of a whitening component, an antiinflammatory component and an antioxidative component, the effect on whitening the skin, improving pigmentation, treating acnes, improving wrinkles, improving skin roughness, improving oily skin, improving dry skin, diminishing the appearance of pores, treating scarring, treating flushing of the face, treating hair loss, promoting hair growth, treating a burn injury, sterilizing the skin, killing mites on the skin and improving skin texture can be enhanced.

## Claims

1. A composition for cosmetic skin treatment **characterized by** comprising a fullerene clathrated in polyvinyl pyrrolidone, wherein said composition further comprises at least one kind of the components listed below:
<1> a nonionic surfactant,
<2> ascorbic acid or a derivative thereof or a salt thereof, and
<3> an ultraviolet protective agent.

2. A composition for cosmetic skin treatment as claimed in claim 1 **characterized in that** the fullerene is C60 fullerene, C70 fullerene or a fullerene mixture containing both.

3. A composition for cosmetic skin treatment as claimed in claim 1 **characterized in that** the pH is from 3 to 10, the total concentration of transition metal compounds is 0.1% or less, and the composition comprises at least one kind of components listed below:
<1> 0.01% to 50% by weight of a nonionic surfactant,
<2> 0.01 % to 20% by weight of an ascorbic acid or a derivative thereof or a salt thereof,
<3> 0.001 to 50% by weight of an ultraviolet protective agent, and
<4> 0.01 % to 10% by weight of a storing stabilizer or an organic acid having a chelating effect or a salt thereof,
wherein the nonionic surfactant is at least one kind selected from a POE sorbitan fatty acid ester such as POE sorbitan monooleate, POE sorbitan monostearate, POE sorbitan monolaurate or POE sorbitan tetraoleate, a POE sorbitol fatty acid ester such as POE sorbitol monolaurate, POE sorbitol monooleate, POE sorbitol pentaoleate or POE sorbitol monostearate, a POE glycerin fatty acid ester such as POE glycerin monostearate or POE glycerin triisostearate, a POE fatty acid ester such as POE monooleate, a POE alkyl ether such as POE lauryl ether, a POE alkylphenyl ether such as POE octylphenyl ether, a POE-POP alkyl ether such as POE-POP cetyl ether, a tetra POE-tetra POE ethylenediamine condensate, POE castor oil, a hardened castor oil derivative, a POE beeswax and lanolin derivative, an alkanolamide such as lauric monoethanolamide, a POE propylene glycol fatty acid ester, a POE alkylamine, a POE fatty acid amide, a sucrose fatty acid ester, a POE nonylphenyl formaldehyde condensate, an alkyl ethoxy dimethylamine oxide, trioleyl phosphate and a polyglycerin fatty acid ester,
wherein the ascorbic acid or the derivative thereof or the salt thereof is a compound represented by the general formula (1) 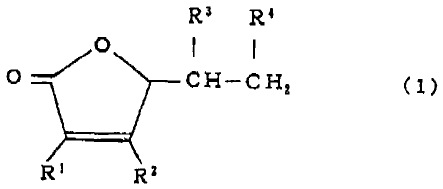 (wherein each of R¹, R², R³ and R⁴ independently denotes a hydroxyl group, an ester group of the hydroxyl group with an inorganic or an organic acid, a glycoside group of the hydroxyl group with a saccharide, a ketal group or an acetal group of adjacent two hydroxyl groups among the hydroxyl groups with a ketone or with an aldehyde, however, R¹ and R² are not a hydroxyl group at the same time) or a salt thereof, or at least one kind selected therefrom,
wherein the ultraviolet protective agent is at least one kind selected from a cinnamic acid-based ultraviolet absorbent such as 2-ethylhexyl p-methoxycinnamate, isopropyl p-methoxycinnamate, p-methoxyhydrocinnamate diethanolamine salt, glyceryl mono-2-ethylhexanoate di-p-methoxycinnamate, octyl methoxycinnamate or methyl diisopropylcinnamate, a benzophenone-based ultraviolet absorbent such as 2-hydroxy-4-methoxybenzophenone, 2-hydroxy-4-methoxybenzophenone-5-sulfuric acid, 2-hydroxy-4-methoxybcnzophenone-5-sodium sulfate, 2,4-dihydroxybenzophenone, 2,2'-dihydroxy-4,4'-dimethoxybenzophenone, 2,2'-dihydroxy-4-methoxy-benzophenone, 2,2',4,4'-tetrahydroxybenzophenone or 2-hydroxy-4-n-octoxybenzophenone, a benzoic acid-based ultraviolet absorbent such as p-aminobenzoic acid, ethyl p-aminobenzoate, butyl p-aminobenzoate, 2-ethylhexyl p-dimethylaminobenzoate, glyceryl p-aminobenzoate or amyl p-aminobenzoate, a salicylic acid-based ultraviolet absorbent such as 2-ethylhexyl salicylate, triethanolamine salicylate, homomenthyl salicylate, dipropylene glycol salicylate, methyl salicylate, ethylene glycol salicylate, phenyl salicylate, aryl salicylate, benzyl salycylate, isopropylbenzyl salicylate or potassium salicylate, a dibenzoylmethane-based ultraviolet absorbent such as 4-t-butyl-4'-methoxydibenzoylmethane, 4-isopropyl-dibenzoylmethane, 4-methoxydibenzoylmethane or 4-t-butyl-4'-hydroxydibenzoylmethane, menthyl-O-amino-benzoate, 2-phenyl-benzimidazole-5-sulfuric acid, 2-phenyl-5-methylbenzoxazole, 3-(4-methylbenzilidene)-camphor, 2-ethylhexyl-2-cyano-3,3-diphenylacrylate, 2-ethyl-2-cyano-3,3 '-diphenylacrytate, 2-(2'-hydroxy-5-methylphenyl) benzotriazole, an anthranilic acid-based ultraviolet absorbent such as menthyl anthranilate, an urocanic acid-based ultraviolet absorbent such as ethyl urocanate, titanium oxide, zirconium oxide, cerium oxide, zinc oxide and a silica-coated metal oxide thereof, and
wherein the storing stabilizer or the organic acid having a chelating effect or the salt thereof is at least one kind selected from the group consisting of erythorbic acid and a salt thereof, dibutylhydroxytoluene, tocopherol and a derivative thereof, porphyrin, butylhydroxyanisole, sodium bisulfite, anhydrous sodium sulfite, gallic acid and a derivative thereof, alanine, sodium hydroxyethyl ethylenediamine triacetate, ethylenediamine tetraacetic acid and a salt thereof, citric acid and a salt thereof, gluconic acid, tartaric acid, phytic acid, sodium polyphosphate and sodium metaphosphate.

4. A composition for cosmetic skin treatment as claimed in either of claims 1 or 3, **characterized in that** it further includes at least one kind of components listed below for an effect on whitening the skin, improving pigmentation, treating acnes, improving wrinkles, improving skin roughness, improving oily skin, improving dry skin, diminishing the appearance of pores, treating scarring, treating flushing of the face, treating hair loss, promoting hair growth, treating a burn injury, sterilizing the skin, killing mites on the skin or improving skin texture is enhanced:
0.01 % to 20% by weight of a whitening component,
0.001 to 10% by weight of an antiinflammatory component, and
0.001 to 10% by weight of an antioxidative component,
wherein the whitening component is at least one kind selected from cysteine, a derivative thereof and a salt thereof, glabridin, glabrene, liquiritin, isoliquiritin, placenta extract, hydroquinone and a derivative thereof, resorcinol and a derivative thereof, and glutathione.

5. A composition for cosmetic skin treatment as claimed in claim 4 **characterized in that** the antiinflammatory component is at least one kind selected from glycyrrhizinic acid, glycyrrhetinic acid, a derivative thereof and a salt thereof, mefenamic acid, phenylbutazone, indomethacin, ibuprofen, ketoprofen, allantoin, guaiazulene, panthenol, a derivative thereof and a salt thereof, ε-aminocapronic acid, diclofenac sodium and tranexamic acid.

6. A composition for cosmetic skin treatment as claimed in any one of claims 4 to 5 **characterized in that** the antioxidative component is at least one kind selected from superoxide dismutase, mannitol, histidine, tryptophan, bilirubin, quercetin, quercitrin, polyphenol, proanthocyanidin, tocotrienol, catechin, a catechin derivative, rutin and a derivative thereof, gallic acid and a derivative thereof, ubiquinone, astaxanthin, carotene, other carotenoids, a derivative thereof and a salt thereof, a vitamin B group, a derivative thereof and a salt thereof, a vitamin D group, a derivative thereof and a salt thereof, a vitamin E group, a derivative thereof and a salt thereof, dibutylhydroxytoluene and butylhydroxyanisole.

7. A composition for cosmetic skin treatment as claimed in any one of claims 1 to 6, wherein the composition is a cream, gel, water-soluble preparation, ointment, emulsion, facial mask, cataplasm, dispersion liquid, solid, paste, mousse or cleansing agent.

8. A composition for cosmetic skin treatment **characterized by** comprising a fullerene clathrated in polyvinyl pyrrolidone, wherein the composition is a cream, gel, ointment, emulsion, facial mask, cataplasm, dispersion liquid, solid, paste, mousse or cleansing agent.

9. A composition for cosmetic skin treatment as claimed in claim 8 **characterized in that** the pH is from 3 to 10, the total concentration of transition metal compounds is 0.1 % or less, and the composition comprises at least one kind of components listed below:
<1> 0.01% to 50% by weight of a nonionic surfactant,
<2> 0.01 % to 20% by weight of an ascorbic acid or a derivative thereof or a salt thereof,
<3> 0.001 to 50% by weight of an ultraviolet protective agent, and
<4> 0.01 % to 10% by weight of a storing stabilizer or an organic acid having a chelating effect or a salt thereof,
wherein the nonionic surfactant is at least one kind selected from a POE sorbitan fatty acid ester such as POE sorbitan monooleate, POE sorbitan monostearate, POE sorbitan monolaurate or POE sorbitan tetraoleate, a POE sorbitol fatty acid ester such as POE sorbitol monolaurate, POE sorbitol monooleate, POE sorbitol pentaoleate or POE sorbitol monostearate, a POE glycerin fatty acid ester such as POE glycerin monostearate or POE glycerin triisostearate, a POE fatty acid ester such as POE monooleate, a POE alkyl ether such as POE lauryl ether, a POE alkylphenyl ether such as POE octylphenyl ether, a POE-POP alkyl ether such as POE-POP cetyl ether, a tetra POE-tetra POE ethylenediamine condensate, POE castor oil, a hardened castor oil derivative, a POE beeswax and lanolin derivative, an alkanolamide such as lauric monoethanolamide, a POE propylene glycol fatty acid ester, a POE alkylamine, a POE fatty acid amide, a sucrose fatty acid ester, a POE nonylphenyl formaldehyde condensate, an alkyl ethoxy dimethylamine oxide, trioleyl phosphate and a polyglycerin fatty acid ester,
wherein the ascorbic acid or the derivative thereof or the salt thereof is a compound represented by the general formula (1) 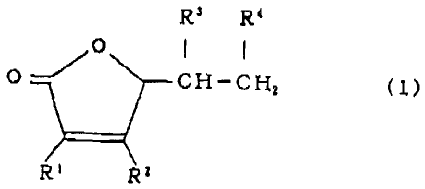 (wherein each of R¹, R², R³ and R⁴ independently denotes a hydroxyl group, an ester group of the hydroxyl group with an inorganic or an organic acid, a glycoside group of the hydroxyl group with a saccharide, a ketal group or an acetal group of adjacent two hydroxyl groups among the hydroxyl groups with a ketone or with an aldehyde, however, R¹ and R² are not a hydroxyl group at the same time) or a salt thereof, or at least one kind selected therefrom,
wherein the ultraviolet protective agent is at least one kind selected from a cinnamic acid-based ultraviolet absorbent such as 2-ethylhexyl p-methoxycinnamate, isopropyl p-methoxycinnamate, p-methoxyhydrocinnamate diethanolamine salt, glyceryl mono-2-ethylhexanoate di-p-metboxycinnamate, octyl methoxycinnamate or methyl diisopropylcinnamate, a benzophenone-based ultraviolet absorbent such as 2-hydroxy-4-methoxybenzophenone, 2-hydroxy-4-methoxybenzophenone-5-sulfuric acid, 2-hydroxy-4-methoxybenzophenone-5-sodium sulfate, 2,4-dihydroxybenzophenone, 2,2'-dihydroxy-4,4'-dimethoxybenzophenone, 2,2'-dihydroxy-4-methoxy-benzophenone, 2,2',4,4'-tetrahydroxybenzophenone or 2-hydroxy-4-n-octoxybenzophenone, a benzoic acid-based ultraviolet absorbent such as p-aminobenzoic acid, ethyl p-aminobenzoate, butyl p-aminobenzoate, 2-ethylhexyl p-dimethylaminobenzoate, glyceryl p-aminobenzoate or amyl p-aminobenzoate, a salicylic acid-based ultraviolet absorbent such as 2-ethylhexyl salicylate, triethanolamine salicylate, homomenthyl salicylate, dipropylene glycol salicylate, methyl salicylate, ethylene glycol salicylate, phenyl salicylate, amyl salicylate, benzyl salycylate, isopropylbenzyl salicylate or potassium salicylate, a dibenzoylmethane-based ultraviolet absorbent such as 4-t-butyl-4'-methoxydibenzoylmethane, 4-isopropyl-dibenzoylmethane, 4-methoxydibenzoylmethane or 4-t-butyl-4'-hydroxydibenzoylmethane, menthyl-O-amino-benzoate, 2-phenyl-benzimidazole-5-sulfuric acid, 2-phenyl-5-methylbenzoxazole, 3-(4-methylbenzilidene)-camphor, 2-ethylhexyl-2-cyano-3,3-diphenylacrylate, 2-ethyl-2-cyano-3,3'-diphenylacrylate, 2-(2'-hydroxy-5-methylphenyl) benzotriazole, an anthranilic acid-based ultraviolet absorbent such as menthyl anthranilate, an urocanic acid-based ultraviolet absorbent such as ethyl urocanate, titanium oxide, zirconium oxide, cerium oxide, zinc oxide and a silica-coated metal oxide thereof, and
wherein the storing stabilizer or the organic acid having a chelating effect or the salt thereof is at least one kind selected from the group consisting of erythorbic acid and a salt thereof, dibutylhydroxytoluene, tocopherol and a derivative thereof, porphyrin, butylhydroxyanisole, sodium bisulfite, anhydrous sodium sulfite, gallic acid and a derivative thereof, alanine, sodium hydroxyethyl ethylenediamine triacetate, ethylenediamine tetraacetic acid and a salt thereof, citric acid and a salt thereof, gluconic acid, tartaric acid, phytic acid, sodium polyphosphate and sodium metaphosphate.

10. A composition for cosmetic skin treatment as claimed in claim 8 or 9, **characterised in that** by further including at least one kind of components listed below, an effect on whitening the skin, improving pigmentation, treating acnes, improving wrinkles, improving skin roughness, improving oily skin, improving dry skin, diminishing the appearance of pores, treating scarring, treating flushing of the face, treating hair loss, promoting hair growth, treating a burn injury, sterilizing the skin, killing mites on the skin or improving skin texture is enhanced:
0.01% to 20% by weight of a whitening component,
0.001 to 10% by weight of an antiinflammatory component, and
0.001 to 10% by weight of an antioxidative component,
wherein the whitening component is at least one kind selected from cysteine, a derivative thereof and a salt thereof, glabridin, glabrene, liquiritin, isoliquiritin, placenta extract, hydroquinone and a derivative thereof, resorcinol and a derivative thereof, and glutathione.

11. A composition for cosmetic skin treatment as claimed in claim 10, wherein the fullerene, antiinflammatory or antioxidative components are as defined in claims 2, 5 or 6.

## Patentansprüche

1. Zusammensetzung zur kosmetischen Hautbehandlung, **dadurch gekennzeichnet, dass** sie ein in Polyvinylpyrrolidon eingeschlossenes Fulleren umfasst, wobei die Zusammensetzung ferner mindestens eine Art der nachfolgend aufgeführten Bestandteile umfasst:
<1> ein nichtionisches Tensid,
<2> Ascorbinsäure oder ein Derivat davon oder ein Salz davon, und
<3> ein UV-Schutzmittel.

2. Zusammensetzung zur kosmetischen Hautbehandlung nach Anspruch 1, **dadurch gekennzeichnet, dass** das Fulleren C60-Fulleren, C70-Fulleren oder eine beides enthaltende Fullerenmischung ist.

3. Zusammensetzung zur kosmetischen Haulbehandlung nach Anspruch 1, **dadurch gekennzeichnet, dass** der pH-Wert von 3 bis 10 beträgt, die Gesamtkonzentration an Übergangsmetallverbindungen 0,1% oder weniger beträgt, und die Zusammensetzung mindestens eine Art der nachfolgend aufgeführten Bestandteile umfasst:
<1> 0,01 bis 50 Gew.-% eines nichtionischen Tensids,
<2> 0,01 bis 20 Gew.-% einer Ascorbinsäure oder eines Derivats davon oder eines Salzes davon,
<3> 0,001 bis 50 Gew.-% eines UV-Schutzmittels, und
<4> 0,01 bis 10 Gew.-% eines Lagerstabilisators oder einer organischen Säure mit einer chelatbildenden Wirkung oder eines Salzes davon,
wobei das nichtionische Tensid mindestens eine Art ist, die aus einem POE-Sorbitanfettsäureester wie zum Beispiel POE-Sorbitanmonooleat, POE-Sorbitanmonostearat, POE-Sorbitanmonolaurat oder POE-Sorbitantetraoleat, einem POE-Sorbitfettsäureester wie zum Beispiel POE-Sorbitmonolaurat, POE-Sorbitmonooleat, POE-Sorbitpentaoleat oder POE-Sorbitmonostearat, einem POE-Glycerinfettsäureester wie zum Beispiel POE-Glycerinmonostearat oder POE-Glycerintriisostearat, einem POE-Fettsäureester wie zum Beispiel POE-Monooleat, einem POE-Alkylether wie zum Beispiel POE-Laurylether, einem POE-Alkylphenylether wie zum Beispiel POE-Octylphenylether, einem POE-POP-Alkylether wie zum Beispiel POE-POP-Cetylether, einem Tetra-POE-tetra-POE-ethylendiaminkondensat, POE-Rizinusöl, einem gehärteten Rizinusölderivat, einem POE-Bienenwachs- und -Lanolinderivat, einem Alkanolamid wie zum Beispiel Laurinsäuremonoethanolamid, einem POE-Propylenglycolfettsäureester, einem POE-Alkylamin, einem POE-Fettsäureamid, einem Saccharosefettsäureester, einem POE-Nonylphenylformaldehydkondensat, einem Alkylethoxydimethylaminoxid, Trioleylphosphat und einem Polyglycerinfettsäureester ausgewählt ist,
wobei die Ascorbinsäure oder das Derivat davon oder das Salz davon eine Verbindung ist, die durch die allgemeine Formel (I) repräsentiert wird: 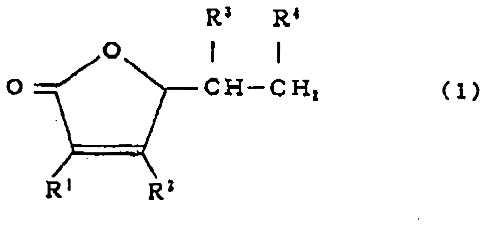 (worin R¹, R², R³ und R⁴ jeweils unabhängig voneinander eine Hydroxylgruppe, eine Estergruppe der Hydroxylgruppe mit einer anorganischen Säure oder einer organischen Säure, eine Glycosidgruppe der Hydroxylgruppe mit einem Saccharid, eine Ketalgruppe oder eine Acetalgruppe von zwei benachbarten Hydroxylgruppen unter den Hydroxylgruppen mit einem Keton oder mit einem Aldehyd bezeichnet, jedoch sind R¹ und R² nicht gleichzeitig eine Hydroxylgruppe) oder ein Salz davon oder mindestens eine daraus ausgewählte Art,
wobei das UV-Schutzmittel mindestens eine Art ist, die aus einem auf Zimtsäure basierenden UV-Absorptionsmittel wie zum Beispiel 2-Ethylhexyl-p-methoxycinnamat, Isopropyl-p-methoxycinnamat, p-Methoxyhydrocinnamatdiethanolaminsalz, Glycerylmono-2-ethylhexanoat-di-p-methoxycinnamat, Octylmethoxycinnamat oder Methyldiisopropylcinnamat, einem auf Benzophenon basierenden UV-Absorptionsmittel wie zum Beispiel 2-Hydroxy-4-methoxybenzophenon, 2-Hydroxy-4-methoxybenzophenon-5-schwefelsäure, 2-Hydroxy-4-methoxybenzophenon-5-natriumsulfat, 2,4-Dihydroxybenzophenon, 2,2'-Dihydroxy-4,4'-dimethoxybenzophenon, 2,2'-Dihydroxy-4-methoxybenzophenon, 2,2',4,4'-Tetrahydroxybenzophenon oder 2-Hydroxy-4-n-octoxybenzophenon, einem auf Benzoesäure basierenden UV-Absorptionsmittel wie zum Beispiel p-Aminobenzoesäure, Ethyl-p-aminobenzoat, Butyl-p-aminobenzoat, 2-Ethylhexyl-p-dimethylaminobenzoat, Glyceryl-p-aminobenzoat oder Amyl-p-aminobenzoat, einem auf Salicylsäure basierenden UV-Absorptionsmittel wie zum Beispiel 2-Ethylhexylsalicylat, Triethanolaminsalicylat, Homomenthylsalicylat, Dipropylenglycolsalicylat, Methylsalicylat, Ethylenglycolsalicylat, Phenylsalicylat, Amylsalicylat, Benzylsalicylat, Isopropylbenzylsalicylat oder Kaliumsalicylat, einem auf Dibenzoylmethan basierenden UV-Absorptionsmittel wie zum Beispiel 4-t-Butyl-4'-methoxydibenzoylmethan, 4-Isopropyl-dibenzoylmethan, 4-Methoxydibenzoylmethan oder 4-t-Butyl-4'-hydroxydibenzoylmethan, Menthyl-O-aminobenzoat, 2-Phenyl-benzimidazol-5-schwefelsäure, 2-Phenyl-5-methylbenzoxazol, 3-(4-Methylbenziliden)-kampfer, 2-Ethylhexyl-2-cyano-3,3-diphenylacrylat, 2-Ethyl-2-cyano-3,3'-diphenylacrylat, 2-(2'-hydroxy-5-methylphenyl)benzotriazol, einem auf Anthranilsäure basierenden UV-Absorptionsmittel wie zum Beispiel Menthylanthranilat, einem auf Urocaninsäure basierenden UV-Absorptionsmittel wie zum Beispiel Ethylurocanat, Titanoxid, Zirconiumoxid, Ceroxid, Zinkoxid und einem silikabeschichten Metalloxid davon ausgewählt ist, und
wobei der Lagerstabilisator oder die organische Säure mit einer chelatbildenden Wirkung oder das Salz davon mindestens eine Art ist, die aus der aus Erythorbinsäure und einem Salz davon bestehenden Gruppe, Dibutylhydroxytoluol, Tocopherol und einem Derivat davon, Porphyrin, Butylhydroxyanisol, Natriumbisulfit, wasserfreiem Natriumsulfit, Gallussäure und einem Derivat davon, Alanin, Natriumhydroxyethyl-ethylendiamintriacetat, Ethylendiamintetraessigsäure und einem Salz davon, Zitronensäure und einem Salz davon, Gluconsäure, Weinsäure, Phytinsäure, Natriumpolyphosphat und Natriummetaphosphat ausgewählt ist.

4. Zusammensetzung zur kosmetischen Hautbehandlung nach Anspruch 1 oder 3, **dadurch gekennzeichnet, dass** sie ferner mindestens eine Art der nachfolgend aufgeführten Bestandteile enthält, damit eine die Haut bleichende Wirkung, die Verbesserung der Pigmentierung, die Behandlung von Akne, die Verringerung von Falten, die Verringerung der Hautrauigkeit, die Verbesserung öliger Haut, die Verbesserung trockener Haut, die Verminderung des Auftretens von Poren, die Behandlung von Narben, die Behandlung einer Rötung des Gesichts, die Behandlung von Haarausfall, die Förderung des Haarwachstums, die Behandlung einer Verbrennung, das Sterilisieren der Haut, das Abtöten von Milben auf der Haut oder die Verbesserung der Hauttextur unterstützt wird:
0,01% bis 20% Gew.-% eines bleichenden Bestandteils,
0,001 bis 10% Gew.-% eines entzündungshemmenden Bestandteils, und
0,001 bis 10 Gew.-% eines oxidationshemmenden Bestandteils,
wobei der bleichende Bestandteil mindestens eine Art ist, die aus Cystein, einem Derivat davon und einem Salz davon, Glabridin, Glabren, Liquiritin, Isoliquiritin, Plazentaextrakt, Hydrochinon und einem Derivat davon, Resorcin und einem Derivat davon sowie Glutathion ausgewählt ist.

5. Zusammensetzung zur kosmetischen Hautbehandlung nach Anspruch 4, **dadurch gekennzeichnet, dass** der entzündungshemmende Bestandteil mindestens eine Art ist, die aus Glyzyrrhizinsäure, Glyzyrrhetinsäure, einem Derivat davon und einem Salz davon, Mefenaminsäure, Phenylbutazon, Indomethacin,
Ibuprofen, Ketoprofen, Allantoin, Guajazulen, Panthenol, einem Derivat davon und einem Salz davon, ε-Aminocapronsäure, Diclofenac-Natrium und Tranexamsäure ausgewählt ist.

6. Zusammensetzung zur kosmetischen Hautbehandlung nach einem der Ansprüche 4 bis 5, **dadurch gekennzeichnet, dass** der oxidationshemmende Bestandteil mindestens eine Art ist, die aus Superoxid-Dismutase, Mannitol, Histidin, Tryptophan, Bilirubin, Quercetin, Quercitrin, Polyphenol, Proanthocyanidin, Tocotrienol, Catechin, einem Catechinderivat, Rutin und einem Derivat davon, Gallussäure und einem Derivat davon, Ubichinon, Astaxanthin, Carotin, anderen Carotinoiden, einem Derivat davon und einem Salz davon, einer Vitamin B-Gruppe, einem Derivat davon und einem Salz davon, einer Vitamin D-Gruppe, einem Derivat davon und einem Salz davon, einer Vitamin E-Gruppe, einem Derivat davon und einem Salz davon, Dibutylhydroxytoluol und Butylhydroxyanisol ausgewählt ist.

7. Zusammensetzung zur kosmetischen Hautbehandlung nach einem der Ansprüche 1 bis 6, wobei die Zusammensetzung eine Creme, ein Gel, ein wasserlösliches Präparat, eine Salbe, eine Emulsion, eine Gesichtsmaske, Kataplasma, eine Dispersionsflüssigkeit, ein Feststoff, eine Paste, ein Schaum oder ein Reinigungsmittel ist.

8. Zusammensetzung zur kosmetischen Hautbehandlung, **dadurch gekennzeichnet, dass** sie ein in Polyvinylpyrrolidon eingeschlossenes Fulleren umfasst, wobei die Zusammensetzung eine Creme, ein Gel, eine Salbe, eine Emulsion, eine Gesichtsmaske, Kataplasma, eine Dispersionsflüssigkeit, ein Feststoff, eine Paste, ein Schaum oder ein Reinigungsmittel ist.

9. Zusammensetzung zur kosmetischen Hautbehandlung nach Anspruch 8, **dadurch gekennzeichnet, dass** der pH-Wert von 3 bis 10 beträgt, die Gesamtkonzentration an Übergangsmetallverbindungen 0,1% oder weniger beträgt, und die Zusammensetzung mindestens eine Art der nachfolgend aufgeführten Bestandteile umfasst:
<1> 0,01 bis 50 Gew.-% eines nichtionischen Tensids,
<2> 0,01 bis 20 Gew.-% einer Ascorbinsäure oder eines Derivats davon oder eines Salzes davon,
<3> 0,001 bis 50 Gew.-% eines UV-Schutzmittels, und
<4> 0,01 bis 10 Gew.-% eines Lagerstabilisators oder einer organischen Säure mit einer chelatbildenden Wirkung oder eines Salzes davon,
wobei das nichtionische Tensid mindestens eine Art ist, die aus einem POE-Sorbitanfettsäureester wie zum Beispiel POE-Sorbitanmonooleat, POE-Sorbitanmonostearat, POE-Sorbitanmonolaurat oder POE-Sorbitantetraoleat, einem POE-Sorbitfettsäureester wie zum Beispiel POE-Sorbitmonolaurat, POE-Sorbitmonooleat, POE-Sorbitpentaoleat oder POE-Sorbitmonostearat, einem POE-Glycerinfettsäureester wie zum Beispiel POE-Glycerinmonostearat oder POE-Glycerintriisostearat, einem POE-Fettsäureester wie zum Beispiel POE-Monooleat, einem POE-Alkylether wie zum Beispiel POE-Laurylether, einem POE-Alkylphenylether wie zum Beispiel POE-Octylphenylether, einem POE-POP-Alkylether wie zum Beispiel POE-POP-Cetylether, einem Tetra-POE-tetra-POE-ethylendiaminkondensat; POE-Rizinusöl, einem gehärteten Rizinusölderivat, einem POE-Bienenwachs- und -Lanolinderivat, einem Alkanolamid wie zum Beispiel Laurinsäuremonoethanolamid, einem POE-Propylenglycolfettsäureester, einem POE-Alkylamin, einem POE-Fettsäureamid, einem Saccharosefettsäureester, einem POE-Nonylphenylformaldehydkondensat, einem Alkylethoxydimethylaminoxid, Trioleylphosphat und einem Polyglycerinfettsäureester ausgewählt ist,
wobei die Ascorbinsäure oder das Derivat davon oder das Salz davon eine Verbindung ist, die durch die allgemeine Formel (I) repräsentiert wird: 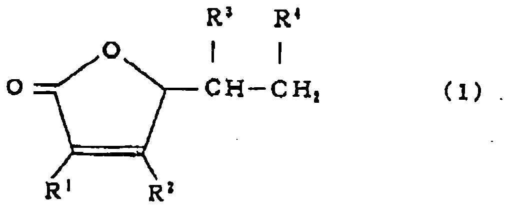 (worin R¹, R², R³ und R⁴ jeweils unabhängig voneinander eine Hydroxylgruppe, eine Estergruppe der Hydroxylgruppe mit einer anorganischen Säure oder einer organischen Säure, eine Glycosidgruppe der Hydroxylgruppe mit einem Saccharid, eine Ketalgruppe oder eine Acetalgruppe von zwei benachbarten Hydroxylgruppen unter den Hydroxylgruppen mit einem Keton oder mit einem Aldehyd bezeichnet, jedoch sind R¹ und R² nicht gleichzeitig eine Hydroxylgruppe) oder ein Salz davon oder mindestens eine daraus ausgewählte Art,
wobei das UV-Schutzmittel mindestens eine Art ist, die aus einem auf Zimtsäure basierenden UV-Absorptionsmittel wie zum Beispiel 2-Ethylhexyl-p-methoxycinnamat, Isopropyl-p-methoxycinnamat, p-Methoxyhydrocinnamatdiethanolaminsalz, Glycerylmono-2-ethylhexanoat-di-p-methoxycinnamat, Octylmethoxycinnamat oder Methyldiisopropylcinnamat, einem auf Benzophenon basierenden UV-Absorptionsmittel wie zum Beispiel 2-Hydroxy-4-methoxybenzophenon, 2-Hydroxy-4-methoxybenzophenon-5-schwefelsäure, 2-Hydroxy-4-methoxybenzophenon-5-natriumsulfat, 2,4-Dihydroxybenzophenon, 2,2'-Dihydroxy-4,4'-dimethoxybenzophenon, 2,2'-Dihydroxy-4-methoxy-benzophenon, 2,2',4,4'-Tetrahydroxybenzophenon oder 2-Hydroxy-4-n-octoxybenzophenon, einem auf Benzoesäure basierenden UV-Absorptionsmittel wie zum Beispiel p-Aminobenzoesäure, Ethyl-p-aminobenzoat, Butyl-p-aminobenzoat, 2-Ethylhexyl-p-dimethylaminobenzoat, Glyceryl-p-aminobenzoat oder Amyl-p-aminobenzoat, einem auf Salicylsäure basierenden UV-Absorptionsmittel wie zum Beispiel 2-Ethylhexylsalicylat, Triethanolaminsalicylat, Homomenthylsalicylat, Dipropylenglycolsalicylat, Methylsalicylat, Ethylenglycolsalicylat, Phenylsalicylat, Amylsalicylat, Benzylsalicylat, Isopropylbenzylsalicylat oder Kaliumsalicylat, einem auf Dibenzoylmethan basierenden UV-Absorptionsmittel wie zum Beispiel 4-t-Butyl-4'-methoxydibenzoylmethan, 4-Isopropyl-dibenzoylmethan, 4-Methoxydibenzoylmethan oder 4-t-Butyl-4'-hydroxydibenzoylmethan, Menthyl-O-aminobenzoat, 2-Phenyl-benzimidazol-5-schwefelsäure, 2-Phenyl-5-methylbenzoxazol, 3-(4-Methylbenziliden)-kampfer, 2-Ethylhexyl-2-cyano-3,3-diphenylacrylat, 2-Ethyl-2-cyano-3,3'-diphenylacrylat, 2-(2'-hydroxy-5-methylphenyl)benzotriazol, einem auf Anthranilsäure basierenden UV-Absorptionsmittel wie zum Beispiel Menthylanthranilat, einem auf Urocaninsäure basierenden UV-Absorptionsmittel wie zum Beispiel Ethylurocanat, Titanoxid, Zirconiumoxid, Ceroxid, Zinkoxid und einem silikabeschichten Metalloxid davon ausgewählt ist, und
wobei der Lagerstabilisator oder die organische Säure mit einer chelatbildenden Wirkung oder das Salz davon mindestens eine Art ist, die aus der aus Erythorbinsäure und einem Salz davon bestehenden Gruppe, Dibutylhydroxytoluol, Tocopherol und einem Derivat davon, Porphyrin, Butylhydroxyanisol, Natriumbisulfit, wasserfreiem Natriumsulfit, Gallussäure und einem Derivat davon, Alanin, Natriumhydroxyethyl-ethylendiamintriacetat, Ethylendiamintetraessigsäure und einem Salz davon, Zitronensäure und einem Salz davon, Gluconsäure, Weinsäure, Phytinsäure, Natriumpolyphosphat und Natriummetaphosphat ausgewählt ist.

10. Zusammensetzung zur kosmetischen Hautbehandlung nach Anspruch 8 oder 9, **dadurch gekennzeichnet, dass** sie ferner mindestens eine der nachfolgend aufgeführten Bestandteile enthält, damit eine die Haut bleichende Wirkung, die Verbesserung der Pigmentierung, die Behandlung von Akne, die Verringerung von Falten, die Verringerung der Hautrauigkeit, die Verbesserung öliger Haut, die Verbesserung trockener Haut, die Verminderung des Auftretens von Poren, die Behandlung von Narben, die Behandlung einer Rötung des Gesichts, die Behandlung von Haarausfall, die Förderung des Haarwachstums, die Behandlung einer Verbrennung, das Sterilisieren der Haut, das Abtöten von Milben auf der Haut oder die Verbesserung der Hauttextur unterstützt wird:
0,01% bis 20% Gew.-% eines bleichenden Bestandteils,
0,001 bis 10% Gew.-% eines entzündungshemmenden Bestandteils, und
0,001 bis 10 Gew.-% eines oxidationshemmenden Bestandteils,
wobei der bleichende Bestandteil mindestens eine Art ist, die aus Cystein, einem Derivat davon und einem Salz davon, Glabridin, Glabren, Liquiritin, Isoliquiritin, Plazentaextrakt, Hydrochinon und einem Derivat davon, Resorcin und einem Derivat davon sowie Glutathion ausgewählt ist.

11. Zusammensetzung zur kosmetischen Hautbehandlung nach Anspruch 10, wobei das Fulleren, der entzündungshemmende oder oxidationshemmende Bestandteil der Definition in Anspruch 2, 5 oder 6 entspricht.

## Revendications

1. Composition de traitement cosmétique de la peau **caractérisée en ce qu'**elle comprend un fullerène clathraté dans de la polyvinylpyrrolidone, dans laquelle ladite composition comprend en outre au moins un type des composants énumérés ci-dessous :
<1> un agent tensioactif non ionique,
<2> de l'acide ascorbique ou un dérivé de celui-ci ou un sel de celui-ci, et
<3> un agent protecteur vis-à-vis des ultraviolets.

2. Composition de traitement cosmétique de la peau selon la revendication 1, **caractérisée en ce que** le fullerène est un fullerène C60, un fullerène C70 ou un mélange de fullerènes contenant les deux.

3. Composition de traitement cosmétique de la peau selon la revendication 1, **caractérisée en ce que** le pH est de 3 à 10, la concentration totale en composés de métal de transition est de 0,1 % ou moins, et la composition comprend au moins un type des composants énumérés ci-dessous :
<1> 0,01 % à 50 % en poids d'un agent tensioactif non ionique,
<2> 0,01 % à 20 % en poids d'un acide ascorbique ou d'un dérivé de celui-ci ou d'un sel de celui-ci,
<3> 0,001 % à 50 % en poids d'un agent protecteur vis-à-vis des ultraviolets, et
<4> 0,01 % à 10 % en poids d'un stabilisateur de stockage ou d'un acide organique ayant un effet chélatant ou un sel de celui-ci,
dans laquelle l'agent tensioactif non ionique est au moins un type choisi parmi un ester d'acide gras de sorbitan POE tel que le monooléate de sorbitan POE, le monostéarate de sorbitan POE, le monolaurate de sorbitan POE ou le tétraoléate de sorbitan POE, un ester d'acide gras de sorbitol POE tel que le monolaurate de sorbitol POE, le monooléate de sorbitol POE, le pentaoléate de sorbitol POE ou le monostéarate de sorbitol POE, un ester d'acide gras de glycérine POE tel que le monostéarate de glycérine POE ou le triisostéarate de glycérine POE, un ester d'acide gras POE tel que le monooléate de POE, un alkyléther POE tel que le lauryléther POE, un alkylphényléther POE tel que l'octyphényléther POE, un alkyléther POE-POP tel que le cétyléther POE-POP, un condensat d'éthylènediamine tétra POE-tétra POE, l'huile de ricin POE, un dérivé d'huile de ricin durci, un dérivé de cire d'abeille et de lanoline POE, un alcanolamide tel que le monoéthanolamide laurique, un ester d'acide gras de propylène glycol POE, une alkylamine POE, un amide d'acide gras POE, un ester d'acide gras de saccharose, un condensat de nonylphénylformaldéhyde POE, un oxyde d'alkyléthoxydiméthylamine, le phosphate de trioléyle et un ester d'acide gras de polyglycérine,
dans laquelle l'acide ascorbique ou le dérivé de celui-ci ou le sel de celui-ci est un composé représenté par la formule générale (1) 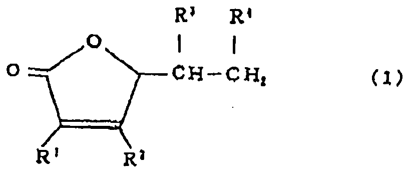 (dans laquelle chacun des R¹, R², R³ et R⁴ désigne indépendamment un groupe hydroxyle, un groupe ester du groupe hydroxyle avec un acide inorganique ou un acide organique, un groupe glycoside du groupe hydroxyle avec un saccharide, un groupe cétal ou un groupe acétal de deux groupes hydroxyle adjacents parmi les groupes hydroxyle avec une cétone ou avec un aldéhyde, toutefois, R¹ et R² ne représentent pas un groupe hydroxyle en même temps) ou un sel de celui-ci, ou au moins un type choisi parmi ceux-ci,
dans laquelle l'agent protecteur vis-à-vis des ultraviolets est au moins un type choisi parmi un absorbeur ultraviolet à base d'acide cinnamique tel que le p-méthoxycinnamate de 2-éthylhexyle, le p-méthoxycinnamate d'isopropyle, le sel de diéthanolamine de p-méthoxyhydrocinnamate, le di-p-méthoxycinnamate de glycéryl mono-2-éthylhexanoate, le méthoxycinnamate d'octyle ou le diisopropylcinnamate de méthyle, un absorbeur ultraviolet à base de benzophénone tel que la 2-hydroxy-4-méthoxybenzophénone, l'acide 2-hydroxy-4-méthoxybenzophénone-5-sulfurique, le sulfate de 2-hydroxy-4-méthoxybenzophénone-5-sodium, la 2,4-dihydroxybenzophénone, la 2,2'-dihydroxy-4,4'-diméthoxybenzophénone, la 2,2'-dihydroxy-4-méthoxy-benzophénone, la 2,2',4,4'-tétrahydroxybenzophénone ou la 2-hydroxy-4-n-octoxybenzophénone, un absorbeur ultraviolet à base d'acide benzoïque tel que l'acide p-aminobenzoïque, le p-aminobenzoate d'éthyle, le p-aminobenzoate de butyle, le p-diméthylaminobenzoate de 2-éthylhexyle, le p-aminobenzoate de glycéryle ou le p-aminobenzoate d'amyle, un absorbeur ultraviolet à base d'acide salicylique tel que le salicylate de 2-éthylhexyle, le salicylate de triéthanolamine, le salicylate d'homomenthyle, le salicylate de dipropylène glycol, le salicylate de méthyle, le salicylate d'éthylène glycol, le salicylate de phényle, le salicylate d'amyle, le salicylate de benzyle, le salicylate d'isopropylbenzyle ou le salicylate de potassium, un absorbeur ultraviolet à base de dibenzoylméthane tel que le 4-t-butyl-4'-méthoxydibenzoylméthane, le 4-isopropyl-dibenzoylméthane, le 4-méthoxydibenzoylméthane ou le 4-t-butyl-4'-hydroxydibenzoylméthane, le O-amino-benzoate de menthyle, l'acide 2-phényl-benzimidazole-5-sulfurique, le 2-phényl-5-méthylbenzoxazole, le 3-(4-méthylbenzilidène)-camphre, l'acrylate de 2-éthylhexyl-2-cyano-3,3-diphényle, l'acrylate de 2-éthyl-2-cyano-3,3'-diphényle, le 2-(2'-hydroxy-5-méthylphényl)benzotriazole, un absorbeur ultraviolet à base d'acide anthranilique tel que l'anthranilate de menthyle, un absorbeur ultraviolet à base d'acide urocanique tel que l'urocanate d'éthyle, l'oxyde de titane, l'oxyde de zirconium, l'oxyde de cérium, l'oxyde de zinc et un oxyde de métal revêtu de silice de celui-ci, et
dans laquelle le stabilisateur de stockage ou l'acide organique ayant un effet chélatant ou le sel de celui-ci est au moins un type choisi dans le groupe constitué par l'acide érythorbique et un sel de celui-ci, le dibutylhydroxytoluène, le tocophérol et un dérivé de celui-ci, la porphyrine, le butylhydroxyanisole, le bisulfite de sodium, le sulfite de sodium anhydre, l'acide gallique et un dérivé de celui-ci, l'alanine, l'hydroxyéthyléthylènediaminetriacétate de sodium, l'acide éthylènediaminetétraacétique et un sel de celui-ci, l'acide citrique et un sel de celui-ci, l'acide gluconique, l'acide tartrique, l'acide phytique, le polyphosphate de sodium et le métaphosphate de sodium.

4. Composition de traitement cosmétique de la peau selon l'une quelconque des revendications 1 ou 3, **caractérisée en ce qu'**elle comporte en outre au moins un type de composant parmi les composants énumérés ci-dessous pour un effet sur le blanchiment de la peau, l'amélioration de la pigmentation, le traitement de l'acné, l'amélioration des rides, l'amélioration de la rugosité de la peau, l'amélioration de la peau grasse, l'amélioration de la peau sèche, la diminution de l'apparition de pores, le traitement de cicatrices, le traitement de rougeurs du visage, le traitement de la perte de cheveux, la promotion de la croissance des cheveux, le traitement d'une lésion par brûlure, une stérilisation de la peau, l'élimination des mites sur la peau ou l'amélioration de la texture de la peau :
0,01 % à 20 % en poids d'un composant de blanchiment,
0,001 à 10 % en poids d'un composant anti-inflammatoire, et
0,001 à 10 % en poids d'un composant antioxydatif,
dans laquelle le composant de blanchiment est au moins un type choisi parmi la cystéine, un dérivé de celle-ci et un sel de celle-ci, la glabridine, le glabrène, la liquiritine, l'isoliquiritine, un extrait de placenta, l'hydroquinone et un dérivé de celle-ci, le résorcinol et un dérivé de celui-ci, et la glutathione.

5. Composition de traitement cosmétique de la peau selon la revendication 4, **caractérisée en ce que** le composant anti-inflammatoire est au moins un type choisi parmi l'acide glycyrrhétinique, l'acide glycyrrhétinique, un dérivé de ceux-ci et un sel de ceux-ci, l'acide méfénamique, la phénylbutazone, l'indométhacine, l'ibuprofène, le kétoprofène, l'allantoïne, le gaïazulène, le panthénol, un dérivé de ceux-ci et un sel de ceux-ci, l'acide ε-aminocapronique, le diclofénac sodium et l'acide tranéxamique.

6. Composition de traitement cosmétique de la peau selon l'une quelconque des revendications 4 à 5, **caractérisée en ce que** le composant antioxydatif est au moins un type choisi parmi la superoxyde dismutase, le mannitol, l'histidine, le tryptophan, la bilirubine, la quercétine, la quercitrine, le polyphénol, la proanthocyanidine, le tocotriénol, la catéchine, un dérivé de catéchine, la rutine et un dérivé de celle-ci, l'acide gallique et un dérivé de celui-ci, l'ubiquinone, l'astaxanthine, le carotène, d'autres caroténoïdes, un dérivé de ceux-ci et un sel de ceux-ci, un membre du groupe de la vitamine B, un dérivé de celui-ci et un sel de celui-ci, un membre du groupe de la vitamine D, un dérivé de celui-ci et un sel de celui-ci, un membre du groupe de la vitamine E, un dérivé de celui-ci et un sel de celui-ci, le dibutylhydroxytoluène et le butylhydroxyanisole.

7. Composition de traitement cosmétique de la peau selon l'une quelconque des revendications 1 à 6, dans laquelle la composition est une crème, un gel, une préparation soluble dans l'eau, une pommade, une émulsion, un masque facial, un cataplasme, un liquide de dispersion, un solide, une pâte, une mousse ou un agent nettoyant.

8. Composition de traitement cosmétique de la peau **caractérisée en ce qu'**elle comprend un fullerène clathraté dans de la polyvinylpyrrolidone, dans laquelle la composition est une crème, un gel, une pommade, une émulsion, un masque facial, un cataplasme, un liquide de dispersion, un solide, une pâte, une mousse ou un agent nettoyant.

9. Composition de traitement cosmétique de la peau selon la revendication 8, **caractérisée en ce que** le pH est de 3 à 10, la concentration totale en composés de métal de transition est de 0,1 % ou moins, et la composition comprend au moins un type des composants énumérés ci-dessous :
<1> 0,01 % à 50 % en poids d'un agent tensioactif non ionique,
<2> 0,01 % à 20 % en poids d'un acide ascorbique ou d'un dérivé de celui-ci ou d'un sel de celui-ci,
<3> 0,001 % à 50 % en poids d'un agent protecteur vis-à-vis des ultraviolets, et
<4> 0,01 % à 10 % en poids d'un stabilisateur de stockage ou d'un acide organique ayant un effet chélatant ou un sel de celui-ci,
dans lequel l'agent tensioactif non ionique est au moins un type choisi parmi un ester d'acide gras de sorbitan POE tel que le monooléate de sorbitan POE, le monostéarate de sorbitan POE, le monolaurate de sorbitan POE ou le tétraoléate de sorbitan POE, un ester d'acide gras de sorbitol POE tel que le monolaurate de sorbitol POE, le monooléate de sorbitol POE, le pentaoléate de sorbitol POE ou le monostéarate de sorbitol POE, un ester d'acide gras de glycérine POE tel que le monostéarate de glycérine POE ou le triisostéarate de glycérine POE, un ester d'acide gras POE tel que le monooléate de POE, un alkyléther POE tel que le lauryléther POE, un alkylphényléther POE tel que l'octyphényléther POE, un alkyléther POE-POP tel que le cétyléther POE-POP, un condensat d'éthylènediamine tétra POE-tétra POE, l'huile de ricin POE, un dérivé d'huile de ricin durci, un dérivé de cire d'abeille et de lanoline POE, un alcanolamide tel que le monoéthanolamide laurique, un ester d'acide gras de propylène glycol POE, une alkylamine POE, un amide d'acide gras POE, un ester d'acide gras de saccharose, un condensat de nonylphénylformaldéhyde POE, un oxyde d'alkyléthoxydiméthylamine, le phosphate de trioléyle et un ester d'acide gras de polyglycérine,
dans laquelle l'acide ascorbique ou le dérivé de celui-ci ou le sel de celui-ci est un composé représenté par la formule générale (1) 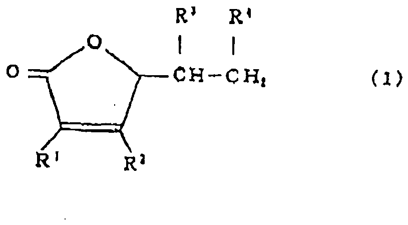 (dans laquelle chacun des R¹, R², R³ et R⁴ désigne indépendamment un groupe hydroxyle, un groupe ester du groupe hydroxyle avec un acide inorganique ou un acide organique, un groupe glycoside du groupe hydroxyle avec un saccharide, un groupe cétal ou un groupe acétal de deux groupes hydroxyle adjacents parmi les groupes hydroxyle avec une cétone ou avec un aldéhyde, toutefois, R¹ et R² ne représentent pas un groupe hydroxyle en même temps) ou un sel de celui-ci, ou au moins un type choisi parmi ceux-ci,
dans laquelle l'agent protecteur vis-à-vis des ultraviolets est au moins un type choisi parmi un absorbeur ultraviolet à base d'acide cinnamique tel que le p-méthoxycinnamate de 2-éthylhexyle, le p-méthoxycinnamate d'isopropyle, le sel de diéthanolamine de p-méthoxyhydrocinnamate, le di-p-méthoxycinnamate de glycéryl mono-2-éthylhexanoate, le méthoxycinnamate d'octyle ou le diisopropylcinnamate de méthyle, un absorbeur ultraviolet à base de benzophénone tel que la 2-hydroxy-4-méthoxybenzophénone, l'acide 2-hydroxy-4-méthoxybenzophénone-5-sulfurique, le sulfate de 2-hydroxy-4-méthoxybenzophénone-5-sodium, la 2,4-dihydroxybenzophénone, la 2,2'-dihydroxy-4,4'-diméthoxybenzophénone, la 2,2'-dihydroxy-4-méthoxy-benzophénone, la 2,2',4,4'-tétrahydroxybenzophénone ou la 2-hydroxy-4-n-octoxybenzophénone, un absorbeur ultraviolet à base d'acide benzoïque tel que l'acide p-aminobenzoïque, le p-aminobenzoate d'éthyle, le p-aminobenzoate de butyle, le p-diméthylaminobenzoate de 2-éthylhexyle, le p-aminobenzoate de glycéryle ou le p-aminobenzoate d'amyle, un absorbeur ultraviolet à base d'acide salicylique tel que le salicylate de 2-éthylhexyle, le salicylate de triéthanolamine, le salicylate d'homomenthyle, le salicylate de dipropylène glycol, le salicylate de méthyle, le salicylate d'éthylène glycol, le salicylate de phényle, le salicylate d'amyle, le salicylate de benzyle, le salicylate d'isopropylbenzyle ou le salicylate de potassium, un absorbeur ultraviolet à base de dibenzoylméthane tel que le 4-t-butyl-4'-méthoxydibenzoylméthane, le 4-isopropyl-dibenzoylméthane, le 4-méthoxydibenzoylméthane ou le 4-t-butyl-4'-hydroxydibenzoylméthane, le O-amino-benzoate de menthyle, l'acide 2-phényl-benzimidazole-5-sulfurique, le 2-phényl-5-méthylbenzoxazole, le 3-(4-méthylbenzilidène)-camphre, l'acrylate de 2-éthylhexyl-2-cyano-3,3-diphényle, l'acrylate de 2-éthyl-2-cyano-3,3'-diphényle, le 2-(2'-hydroxy-5-méthylphényl)benzotriazole, un absorbeur ultraviolet à base d'acide anthranilique tel que l'anthranilate de menthyle, un absorbeur ultraviolet à base d'acide urocanique tel que l'urocanate d'éthyle, l'oxyde de titane, l'oxyde de zirconium, l'oxyde de cérium, l'oxyde de zinc et un oxyde de métal revêtu de silice de celui-ci, et
dans laquelle le stabilisateur de stockage ou l'acide organique ayant un effet chélatant ou le sel de celui-ci est au moins un type choisi dans le groupe constitué par l'acide érythorbique et un sel de celui-ci, le dibutylhydroxytoluène, le tocophérol et un dérivé de celui-ci, la porphyrine, le butylhydroxyanisole, le bisulfite de sodium, le sulfite de sodium anhydre, l'acide gallique et un dérivé de celui-ci, l'alanine, l'hydroxyéthyléthylènediaminetriacétate de sodium, l'acide éthylènediaminetétraacétique et un sel de celui-ci, l'acide citrique et un sel de celui-ci, l'acide gluconique, l'acide tartrique, l'acide phytique, le poly(phosphate) de sodium et le métaphosphate de sodium.

10. Composition de traitement cosmétique de la peau selon la revendication 8 ou 9, **caractérisée en ce qu'**elle comporte en outre au moins un type de composants énumérés ci-dessous, pour renforcer un effet sur le blanchiment de la peau, l'amélioration de la pigmentation, le traitement de l'acné, l'amélioration des rides, l'amélioration de la rugosité de la peau, l'amélioration de la peau grasse, l'amélioration de la peau sèche, la diminution de l'apparition de pores, le traitement de cicatrices, le traitement de rougeurs du visage, le traitement de la perte de cheveux, la promotion de la croissance des cheveux, le traitement d'une lésion par brûlure, une stérilisation de la peau, l'élimination des mites sur la peau ou l'amélioration de la texture de la peau :
0,01 % à 20 % en poids d'un composant de blanchiment,
0,001 à 10 % en poids d'un composant anti-inflammatoire, et
0,001 à 10 % en poids d'un composant antioxydatif,
dans laquelle le composant de blanchiment est au moins un type choisi parmi la cystéine, un dérivé de celle-ci et un sel de celle-ci, la glabridine, le glabrène, la liquiritine, l'isoliquiritine, un extrait de placenta, l'hydroquinone et un dérivé de celle-ci, le résorcinol et un dérivé de celui-ci, et la glutathione.

11. Composition de traitement cosmétique de la peau selon la revendication 10, dans laquelle le fullerène, les composants anti-inflammatoires ou antioxydatifs sont tels que définis dans les revendications 2, 5 ou 6.
